# EUROPÄISCHE PATENTSCHRIFT

(11) **EP 3 293 558 B1**
(45) Veröffentlichungstag und Bekanntmachung des Hinweises auf die Patenterteilung: **10.05.2023**
(21) Anmeldenummer: 17190167.1
(22) Anmeldetag: 08.09.2017
(51) Int. Cl.: G02B 21/22, A61B 1/00, G03B 35/08

(54) **VORRICHTUNG ZUM ERFASSEN EINES STEREOBILDS**
DEVICE FOR RECORDING A STEREO IMAGE
DISPOSITIF DE DÉTECTION D'UNE IMAGE STÉRÉO

(30) Priorität: 09.09.2016 DE 102016117024
(43) Veröffentlichungstag der Anmeldung: 14.03.2018
(73) Patentinhaber: Karl Storz SE & Co. KG, 78532 Tuttlingen (DE)
(72) Erfinder: Bechtel, Christin, 78532 Tuttlingen (DE)
(74) Vertreter: Wimmer, Stephan

(56) Entgegenhaltungen:
- EP-A1- 2 472 318
- EP-A1- 2 597 502
- EP-A2- 2 493 203
- WO-A1-94/10604
- JP-A- H08 304 943
- KR-A- 20130 061 322
- US-A- 4 879 596
- US-A- 6 002 534
- US-A1- 2012 140 044

## Beschreibung

Die vorliegende Erfindung ist auf ein Stereo-Exoskop, ein Stereo-Operationsmikroskop, ein Stereo-Endoskop und andere Vorrichtungen zum Erfassen eines Stereobilds und auf ein Verfahren zum Justieren einer Vorrichtung zum Erfassen eines Stereobilds bezogen.

Bei der Darstellung von Stereobildern hat die Schärfentiefe einen wesentlichen Einfluss auf die Qualität des räumlichen Eindrucks, der beim Betrachter entsteht. Eine Mindest-Schärfentiefe ist erforderlich, um einen räumlichen bzw. dreidimensionalen Eindruck hervorzurufen. Andererseits kann eine sehr große Schärfentiefe eine gleichzeitige scharfe Darstellung von Objekten in sehr unterschiedlichen Entfernungen und deshalb mit sehr unterschiedlichen Disparitäten bewirken und deshalb als unangenehm wahrgenommen werden.

In vielen Fällen wird versucht, mit einer großen Aperturblende ein hoch aufgelöstes Bild zu erzeugen. Je größer die Aperturblende, desto geringer ist die Schärfentiefe.

In US 2012/0140044 A1 ist eine Erfassung eines Stereobilds mittels zweier Flüssigkristalllinsen, deren Brennweiten unabhängig von einander einstellbar sind, beschrieben (Absätze [0057], [0083]). Die beiden Flüssigkristalllinsen sind so eingestellt, dass ein betrachtetes Objekt nahe der distalen Grenze des Schärfentiefenbereichs des für das linke Auge erfassten Bilds und nahe der proximalen Grenze des Schärfentiefenbereichs des für das rechte Auge erfassten Bilds liegt (Fig. 14C).

In US 2011/0018972 A1 ist eine stereoskopische Abbildungsvorrichtung mit zwei Objektiven beschrieben, wobei eine unterschiedliche Fokussierung beider Objektive vermieden werden soll (Absätze [0011], [0013], [0014]).

In US 2015/0156478 A1) ist eine 3D-Darstellung erwähnt (Absatz [0156]).

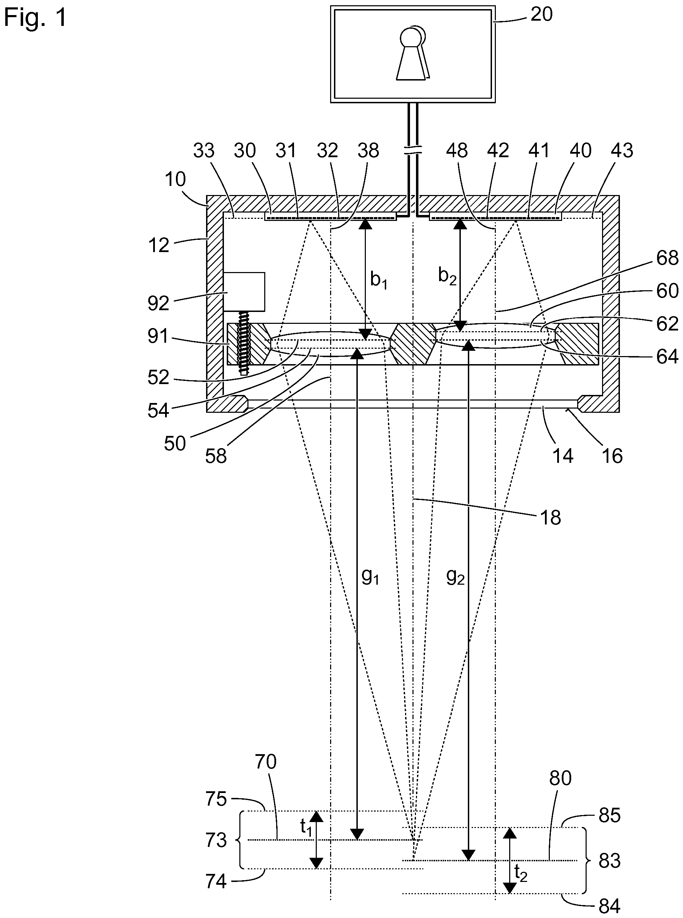

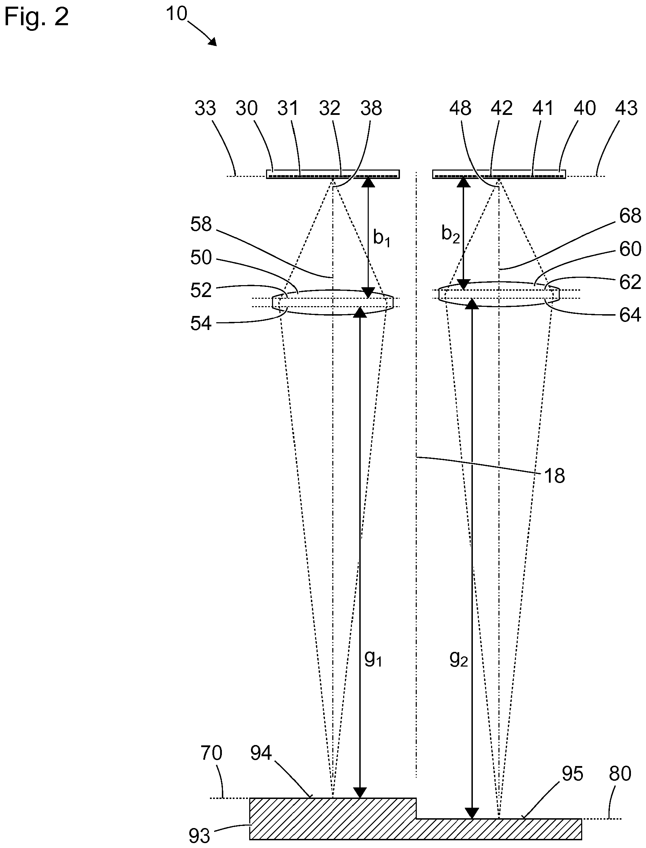

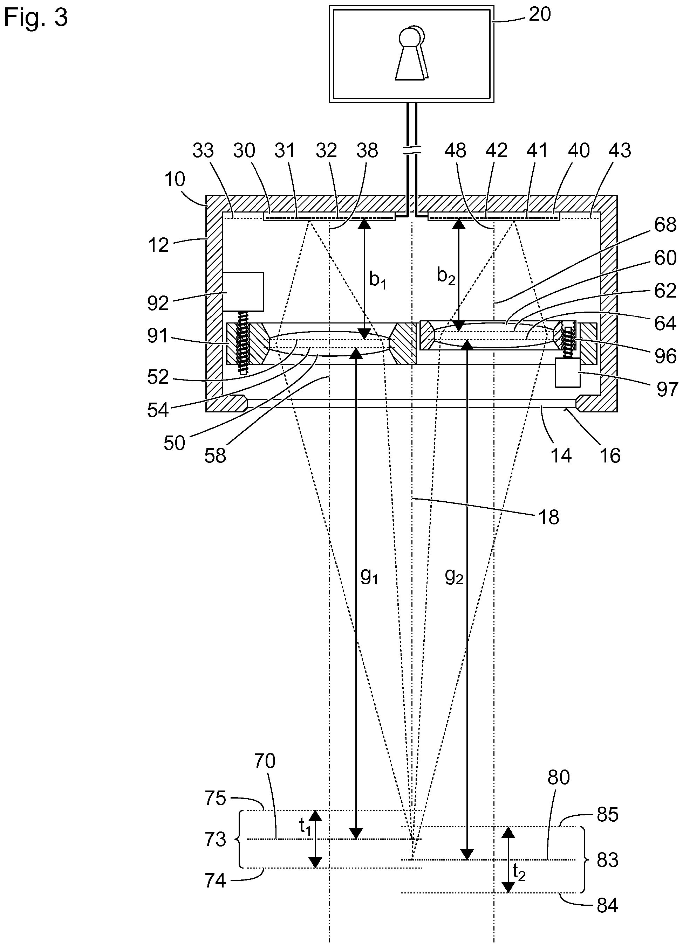

In EP 2 597 502 A1 sind eine Bilderfassungsvorrichtung ("imaging device") und ein Verfahren zu deren Steuerung beschrieben (Absätze [0001], [0029], Figur 1). Die Bilderfassungsvorrichtung 100 umfasst eine Bilderfassungseinheit 200 für das linke Auge ("left-eye imaging unit") 200 und eine Bilderfassungseinheit 300 für das rechte Auge ("right-eye imaging unit") 300 mit je einer Zoomlinse ("zoom lens") 211, 311, einer Fokuslinse ("focus lens") 213, 313 und einem Bilderfassungselement ("imaging element") 250, 350 (Absätze [0034], [0035], Figur 2). Eine Fokussteuerungseinheit ("focus control unit") 123 kann die Bilderfassungseinheiten 200, 300 für das linke Auge und für das rechte Auge so steuern, dass die Schärfentiefenbereiche ("range of depth of field") des für das linke Auge bestimmten Bilds und des für das rechte Auge bestimmten Bilds unterschiedlich sein können (Absatz [0066], Figur 3). Die Schärfentiefenbereiche können an einander anschließen ("be continuous with") oder überlappen (ebd., Absatz [0081]).

In US 4,879,596 ist eine Stereokameravorrichtung ("stereoscopic camera apparatus") beschrieben (Spalte 1, Zeilen 9½ bis 13½). Die Stereokameravorrichtung umfasst zwei Kameras 20a, 20b an einem Hauptrahmen ("main frame") 24 (Spalte 3, Zeilen 8 bis 12; Figur 3A). Jede der beiden Kameras 20a, 20b kann in einer vertikalen Richtung geschwenkt ("pivotally adjusted in the vertical direction"; Spalte 3, Zeilen 32 bis 34 und Zeilen 45 bis 46) und um ihre optische Achse geschwenkt ("pivotally adjusted about its optical axis"; Spalte 3, Zeilen 62 bis 65, und Spalte 4, Zeilen 7 bis 9) werden.

In EP 2 472 318 A1 ist eine parallele Einstellung der optischen Achsen zweier Objektivtuben ("lens barrel") einer Stereoabbildungsvorrichtung ("stereoscopic imaging apparatus") beschrieben (Absätze [0001], [0010], [0105]).

In WO 94/10604 A1 ist eine Stereoabbildungsvorrichtung ("stereoscopic imaging apparatus") mit zwei identischen Kameras 10, 11, die unabhängig von einander azimuthal rotierbar sind, beschrieben (Seite 4, Zeilen 25 bis 35). Ein formschlüssiges Blendensteuerungs- und Fokussierungssystem ("positive locking aperture control and focussing system") ermöglicht eine identische Einstellung von Fokus und Belichtung bei beiden Kameras 10, 11 (Seite 5, Zeilen 9 bis 12).

Eine Aufgabe der vorliegenden Erfindung besteht darin, eine verbesserte Vorrichtung zum Erfassen eines Stereobilds und ein verbessertes Verfahren zum Justieren einer Vorrichtung zum Erfassen eines Stereobilds zu schaffen.

Diese Aufgabe wird durch die Gegenstände der unabhängigen Ansprüche gelöst. Weiterbildungen sind in den abhängigen Ansprüchen angegeben.

Eine nicht erfindungsgemäße Vorrichtung zum Erfassen eines Stereobilds umfasst einen ersten Bildsensor mit einer ersten lichtempfindlichen Schicht zum Erfassen eines ersten Bilds, einen zweiten Bildsensor mit einer zweiten lichtempfindlichen Schicht zum Erfassen eines zweiten Bilds, ein erstes Objektiv, das Punkte, die in einer ersten Fläche liegen, scharf auf die erste lichtempfindliche Schicht des ersten Bildsensors abbildet, und ein zweites Objektiv, das Punkte, die in einer zweiten Fläche liegen, scharf auf die zweite lichtempfindliche Schicht des zweiten Bildsensors abbildet, wobei in der Mitte eines Bereichs, der sowohl von dem ersten Objektiv auf den ersten Bildsensor als auch von dem zweiten Objektiv auf den zweiten Bildsensor abgebildet wird, die erste Fläche und die zweite Fläche voneinander beabstandet sind.

Eine nicht erfindungsgemäße Vorrichtung zum Erfassen eines Stereobilds umfasst einen ersten Bildsensor mit einer ersten lichtempfindlichen Schicht zum Erfassen eines ersten Bilds, einen zweiten Bildsensor mit einer zweiten lichtempfindlichen Schicht zum Erfassen eines zweiten Bilds, ein erstes Objektiv mit einer ersten bildseitigen Hauptebene, das Punkte, die in einer ersten Fläche liegen, scharf auf die erste lichtempfindliche Schicht des ersten Bildsensors abbildet, und ein zweites Objektiv mit einer zweiten bildseitigen Hauptebene, das Punkte, die in einer zweiten Fläche liegen, scharf auf die zweite lichtempfindliche Schicht des zweiten Bildsensors abbildet, wobei zumindest entweder eine erste Gerade durch die Mitte der ersten lichtempfindlichen Schicht des ersten Bildsensors und durch die Mitte der zweiten lichtempfindlichen Schicht des zweiten Bildsensors oder eine zweite Gerade durch die Mitte der ersten bildseitigen Hauptebene des ersten Objektivs und durch die Mitte der zweiten bildseitigen Hauptebene des zweiten Objektivs nicht orthogonal zu einer Hauptblickachse der Vorrichtung sind.

Die Mitte einer lichtempfindlichen Schicht eines Bildsensors ist insbesondere der Flächenschwerpunkt desjenigen Bereichs der lichtempfindlichen Schicht, aus dem bei der vorgesehenen Verwendung der Vorrichtung tatsächlich Bilddaten ausgelesenen und weiterverarbeitet werden. Die Mitte einer Hauptebene eines Objektivs ist der Schnittpunkt der Hauptebene mit der optischen Achse des Objektivs.

Diese Anordnung bewirkt insbesondere, dass die erste Fläche und die zweite Fläche beabstandet oder in Richtung parallel zu der Hauptblickachse der Vorrichtung versetzt sind.

Eine nicht erfindungsgemäße Vorrichtung zum Erfassen eines Stereobilds umfasst einen ersten Bildsensor mit einer ersten lichtempfindlichen Schicht zum Erfassen eines ersten Bilds, einen zweiten Bildsensor mit einer zweiten lichtempfindlichen Schicht zum Erfassen eines zweiten Bilds, ein erstes Objektiv mit einer ersten bildseitigen Hauptebene, das Punkte, die in einer ersten Fläche liegen, scharf auf die erste lichtempfindliche Schicht des ersten Bildsensors abbildet, und ein zweites Objektiv mit einer zweiten bildseitigen Hauptebene, das Punkte, die in einer zweiten Fläche liegen, scharf auf die zweite lichtempfindliche Schicht des zweiten Bildsensors abbildet, wobei der erste Bildsensor und das erste Objektiv nicht spiegelsymmetrisch zu dem zweiten Bildsensor und dem zweiten Objektiv angeordnet sind.

Insbesondere sind der erste Bildsensor und das erste Objektiv nicht spiegelsymmetrisch zu dem zweiten Bildsensor und dem zweiten Objektiv.

Die asymmetrische Anordnung bewirkt insbesondere, dass die erste Fläche und die zweite Fläche beabstandet oder in Richtung parallel zu der Hauptblickachse der Vorrichtung versetzt sind.

Eine nicht erfindungsgemäße Vorrichtung zum Erfassen eines Stereobilds umfasst einen ersten Bildsensor mit einer ersten lichtempfindlichen Schicht zum Erfassen eines ersten Bilds, einen zweiten Bildsensor mit einer zweiten lichtempfindlichen Schicht zum Erfassen eines zweiten Bilds, ein erstes Objektiv mit einer ersten bildseitigen Hauptebene, das Punkte, die in einer ersten Fläche liegen, scharf auf die erste lichtempfindliche Schicht des ersten Bildsensors abbildet, und ein zweites Objektiv mit einer zweiten bildseitigen Hauptebene, das Punkte, die in einer zweiten Fläche liegen, scharf auf die zweite lichtempfindliche Schicht des zweiten Bildsensors abbildet, wobei keine Symmetrieebene existiert, zu der sowohl der erste Bildsensor und der zweite Bildsensor als auch das erste Objektiv und das zweite Objektiv spiegelsymmetrisch angeordnet sind.

Insbesondere ist die Anordnung des ersten Bildsensors und des ersten Objektivs nicht spiegelsymmetrisch zu der Anordnung des zweiten Bildsensors und des zweiten Objektivs.

Die asymmetrische Anordnung bewirkt insbesondere, dass die erste Fläche und die zweite Fläche beabstandet oder in Richtung parallel zu der Hauptblickachse der Vorrichtung versetzt sind.

Eine nicht erfindungsgemäße Vorrichtung zum Erfassen eines Stereobilds umfasst einen ersten Bildsensor mit einer ersten lichtempfindlichen Schicht zum Erfassen eines ersten Bilds, einen zweiten Bildsensor mit einer zweiten lichtempfindlichen Schicht zum Erfassen eines zweiten Bilds, ein erstes Objektiv mit einer ersten bildseitigen Hauptebene, das Punkte, die in einer ersten Fläche liegen, scharf auf die erste lichtempfindliche Schicht des ersten Bildsensors abbildet, und ein zweites Objektiv mit einer zweiten bildseitigen Hauptebene, das Punkte, die in einer zweiten Fläche liegen, scharf auf die zweite lichtempfindliche Schicht des zweiten Bildsensors abbildet, wobei zumindest entweder die erste lichtempfindliche Schicht des ersten Bildsensors und die zweite lichtempfindliche Schicht des zweiten Bildsensors in zwei parallelen und von einander beabstandeten Ebenen angeordnet sind oder die erste bildseitige Hauptebene des ersten Objektivs und die zweite bildseitige Hauptebene des zweiten Objektivs parallel und von einander beabstandet sind.

Diese Anordnung bewirkt insbesondere, dass die erste Fläche und die zweite Fläche beabstandet oder in Richtung parallel zu der Hauptblickachse der Vorrichtung versetzt sind.

Eine erfindungsgemäße Vorrichtung zum Erfassen eines Stereobilds umfasst einen ersten Bildsensor mit einer ersten lichtempfindlichen Schicht zum Erfassen eines ersten Bilds, einen zweiten Bildsensor mit einer zweiten lichtempfindlichen Schicht zum Erfassen eines zweiten Bilds, ein erstes Objektiv mit einer ersten bildseitigen Hauptebene, das Punkte, die in einer ersten Fläche liegen, scharf auf die erste lichtempfindliche Schicht des ersten Bildsensors abbildet, und ein zweites Objektiv mit einer zweiten bildseitigen Hauptebene, das Punkte, die in einer zweiten Fläche liegen, scharf auf die zweite lichtempfindliche Schicht des zweiten Bildsensors abbildet, wobei der Abstand der ersten bildseitigen Hauptebene des ersten Objektivs von der ersten lichtempfindlichen Schicht des ersten Bildsensors und der Abstand der zweiten bildseitigen Hauptebene des zweiten Objektivs von der zweiten lichtempfindlichen Schicht des zweiten Bildsensors verschieden sind, so dass die erste Fläche und die zweite Fläche beabstandet oder in Richtung parallel zu der Hauptblickachse der Vorrichtung versetzt sind.

Die Vorrichtung ist insbesondere ein Stereo-Operationsmikroskop, ein anderes Stereomikroskop, ein Stereo-Exoskop oder ein Stereo-Endoskop für Anwendungen in der Medizin und/oder für technische oder andere nicht medizinische Anwendungen. Alternativ kann die Vorrichtung Bestandteil eines Stereo-Operationsmikroskops, eines anderen Stereomikroskops, eines Stereo-Exoskops oder eines Stereo-Endoskops für Anwendungen in der Medizin und/oder für technische oder andere nicht medizinische Anwendungen sein.

Der erste Bildsensor und das erste Objektiv bilden gemeinsam insbesondere eine erste monokulare Kamera. Der zweite Bildsensor und das zweite Objektiv bilden zusammen insbesondere eine zweite monokulare Kamera. Zwischen beiden monokularen Kameras muss nicht, kann jedoch eine teilweise oder vollständige räumliche und/oder konstruktive Trennung bestehen. Jede der beiden monokularen Kameras kann mehrere Bildsensoren zum Wandeln von Licht in unterschiedlichen Wellenlängenbereichen in elektrische oder andere Signale umfassen.

Der erste Bildsensor und der zweite Bildsensor können als zwei separate, optional auch voneinander räumlich beabstandete Bauelemente (beispielsweise CCD oder CMOS oder andere Halbleiterbauelemente) ausgebildet sein. Alternativ können der erste Bildsensor durch einen ersten Bereich eines Bauelements und der zweite Bildsensor durch einen zweiten Bereich desselben Bauelements gebildet sein, wobei der erste Bereich und der zweite Bereich des Bauelements insbesondere voneinander beabstandet sind.

Der erste Bildsensor ist insbesondere zum Erfassen eines ersten Bilds, das - optional nach Verstärkung, Bearbeitung und Aufbereitung - zur Betrachtung mit dem linken Auge eines Betrachters vorgesehen ist, ausgebildet. Der zweite Bildsensor ist insbesondere zur Erfassung eines zweiten Bilds, das zur gleichzeitigen Betrachtung mit dem rechten Auge des Betrachters vorgesehen ist, ausgebildet. Alternativ können das erste Bild zur Betrachtung mit dem rechten Auge und das zweite Bild zur Betrachtung mit dem linken Auge vorgesehen sein.

Die mittels der Bildsensoren erfassten Bilder können gleichzeitig oder abwechselnd durch ein und denselben Bildschirm mit unterschiedlichen Farben oder unterschiedlichen Polarisationen oder abwechselnd in unterschiedlichen Zeitintervallen wiedergegeben werden. Dabei wird insbesondere durch eine vom Betrachter zu tragende Brille sichergestellt, dass sein linkes Auge nur das erste Bild und sein rechtes Auge nur das zweite Bild sehen kann (oder umgekehrt). Alternativ kann beispielsweise für jedes Auge eines Betrachters ein zugeordneter Bildschirm vorgesehen sein.

Das erste Objektiv und das zweite Objektiv können jeweils eine oder mehrere Linsen oder andere optische Elemente aufweisen, wobei jedes optische Element eine oder mehrere gekrümmte Licht brechende oder reflektierende Flächen und/oder einen ortsabhängig variierenden Brechungsindex (beispielsweise GRIN Linsen) aufweist oder Licht beugt. Das erste Objektiv und das zweite Objektiv weisen insbesondere Aperturblenden mit gleichem Durchmesser und/oder gleicher Fläche auf. Das erste Objektiv und das zweite Objektiv sind insbesondere baugleich.

Die lichtempfindlichen Schichten der Bildsensoren sind insbesondere so dünn, dass sie im vorliegenden technischen Kontext als zweidimensionale Objekte, nämlich Flächen, insbesondere ebene Flächen, betrachtet werden können. Jedes Objektiv bildet - von Beugungseffekten und anderen Abbildungsfehlern abgesehen - jeden ersten Punkt im Objekt- bzw. Gegenstandsraum auf einen zweiten Punkt im Bildraum ab. Zu jedem punktförmigen oder flächigen Objekt auf der Objektseite korrespondiert also ein punktförmiges oder flächiges Bild auf der Bildseite des Objektivs. Das Abbildungsverhalten vieler Objektive wird zumindest näherungsweise durch die Linsengleichung 1/g+ 1/b= 1/f beschrieben, wobei g die Gegenstands- oder Objektweite (der Abstand eines Objekts von einer objektsseitigen Hauptebene des Objektivs), b die Bildweite (der Abstand des Bilds des Objekt von einer bildseitigen Hauptebene des Objektivs) und f die Brennweite des Objektivs sind. Die Brennweite f und die Positionen der objektseitigen und bildseitigen Hauptebenen sind Kenngrößen jedes Objektivs.

Die erste Fläche ist die - idealerweise ebene - Objektfläche, die zu der ersten lichtempfindlichen Schicht des ersten Bildsensors korrespondiert, die also durch das erste Objektiv scharf in die erste lichtempfindliche Schicht des ersten Bildsensors abgebildet wird. Das erste Objektiv erzeugt von einem punktförmigen Objekt, das nicht in der ersten Fläche liegt, ein nicht punktförmiges Bild in der ersten lichtempfindlichen Schicht des ersten Bildsensors. Das nicht punktförmige Bild wird auch als Zerstreuungs- oder Unschärfekreis bezeichnet. Die Größe des nicht punktförmigen Bilds ist abhängig von dem Abstand des punktförmigen Objekts von der ersten Fläche.

Die zweite Fläche ist die - im Idealfall ebene - Objektfläche, die zu der zweiten lichtempfindlichen Schicht des zweiten Bildsensors korrespondiert, die also durch das zweite Objektiv scharf in die zweite lichtempfindliche Schicht des zweiten Bildsensors abgebildet wird. Das zweite Objektiv erzeugt von einem punktförmigen Objekt, der nicht in der zweiten Fläche liegt, ein nicht punktförmiges Bild in der zweiten lichtempfindlichen Schicht des zweiten Bildsensors. Die Grö-ße des nicht punktförmigen Bilds ist abhängig von dem Abstand des punktförmigen Objekts von der zweiten Fläche.

Wenn die erste Fläche, die scharf in die erste lichtempfindliche Schicht des ersten Bildsensors abgebildet wird, und die zweite Fläche, die scharf in die zweite lichtempfindliche Schicht des zweiten Bildsensors abgebildet wird, jeweils eben, zu einander parallel und von einander beabstandet sind, schneiden sie einander nicht. Wenn die erste Fläche und die zweite Fläche jeweils nicht eben sind, schneiden sie einander in der Regel auch dann, wenn sie gegeneinander versetzt angeordnet sind. Die Schnittlinie liegt dann aber in der Regel nicht auf der Hauptblickachse der Vorrichtung.

Die Mitte des Bereichs, der sowohl von dem ersten Objektiv auf den ersten Bildsensor als auch von dem zweiten Objektiv auf den zweiten Bildsensor abgebildet wird, liegt insbesondere auf einer Geraden, die die Hauptblickachse der Vorrichtung definiert.

Im Falle paralleler optischer Achsen der Objektive sind die Hauptblickrichtung die Richtung der optischen Achsen und die Hauptblickachse die zu den optischen Achsen der Objektive parallele Gerade, die in derjenigen Ebene liegt, die beide optische Achsen enthält, und die zu beiden optischen Achsen gleiche Abstände aufweist. In diesem Fall ist die Mitte des Bereichs, der sowohl von dem ersten Objektiv auf den ersten Bildsensor als auch von dem zweiten Objektiv auf den zweiten Bildsensor abgebildet wird, insbesondere die Mitte zwischen den optischen Achsen der Objektive.

Im Falle sich schneidender optischer Achsen ist die Hauptblickachse der Vorrichtung insbesondere die Richtung derjenigen winkelhalbierenden Geraden in der durch die optischen Achsen der Objektive definierten Ebene, die mit beiden optischen Achsen gleiche Winkel einschließt, und die zwischen den Objektiven liegt.

Im Falle windschiefer (d. h. weder paralleler noch sich schneidender) optischer Achsen der Objektive ist die Hauptblickachse diejenige Gerade, die mit beiden optischen Achsen minimale Winkel einschließt, und die so angeordnet ist, dass für jede Ebene orthogonal zu der Geraden der Schnittpunkt der Geraden mit dieser Ebene in der Mitte zwischen den Schnittpunkten der optischen Achsen mit dieser Ebene liegt.

Wenn die bildseitige Hauptebene des ersten Objektivs nicht parallel zu der ersten lichtempfindlichen Schicht des ersten Bildsensors ist, ist der Abstand der bildseitigen Hauptebene des ersten Objektivs von der ersten lichtempfindlichen Schicht des ersten Bildsensors der Abstand zwischen dem Schnittpunkt der optischen Achse des ersten Objektivs mit der bildseitigen Hauptebene des ersten Objektivs und dem Schnittpunkt der optischen Achse des ersten Objektivs mit der ersten lichtempfindlichen Schicht des ersten Bildsensors.

Wenn die bildseitige Hauptebene des zweiten Objektivs nicht parallel zu der zweiten lichtempfindlichen Schicht des zweiten Bildsensors ist, ist der Abstand der bildseitigen Hauptebene des zweiten Objektivs von der zweiten lichtempfindlichen Schicht des zweiten Bildsensors der Abstand zwischen dem Schnittpunkt der optischen Achse des zweiten Objektivs mit der zweiten bildseitigen Hauptebene des zweiten Objektivs und dem Schnittpunkt der optischen Achse des zweiten Objektivs mit der zweiten lichtempfindlichen Schicht des zweiten Bildsensors.

Das erste Objektiv und das zweite Objektiv können eine sehr hochwertige bzw. nur vergleichsweise kleine Abbildungsfehler aufweisende Abbildung ermöglichen. Die Fokussierung auf unterschiedliche Flächen durch unterschiedliche Abstände der bildseitigen Hauptebenen von den lichtempfindlichen Schichten der Bildsensoren stellt eine rein mechanische Aufgabe dar, die dauerhaft und insbesondere mechanisch robust gelöst werden kann. Eine Rücksichtnahme auf - insbesondere thermisch - empfindliche Flüssigkristalllinsen ist nicht erforderlich, die aufwendige elektronische Ansteuerung von Flüssigkristalllinsen entfällt.

Bei einer Vorrichtung, wie sie hier beschrieben ist, weisen das erste Objektiv und das zweite Objektiv insbesondere die gleiche Brennweite auf.

Das erste Objektiv und das zweite Objektiv weisen insbesondere nominell bzw. innerhalb der Fertigungstoleranzen die gleiche Brennweite auf. Ein Versatz bzw. ein Abstand der ersten Fläche, in der Punkte liegen, die durch das erste Objektiv scharf auf die erste lichtempfindliche Schicht des ersten Bildsensors abgebildet werden, von der zweiten Fläche, in der Punkte liegen, die durch das zweite Objektiv scharf auf die zweite lichtempfindliche Schicht des zweiten Bildsensors abgebildet werden, ist allein durch unterschiedliche Bildweiten bzw. Abstände der bildseitigen Hauptebenen von den zugeordneten lichtempfindlichen Schichten realisierbar.

Bei einer Vorrichtung, wie sie hier beschrieben ist, sind insbesondere die erste bildseitige Hauptebene des ersten Objektivs und die zweite bildseitige Hauptebene des zweiten Objektivs parallel und voneinander beabstandet.

Bei einer Vorrichtung, wie sie hier beschrieben ist, liegen die lichtempfindlichen Schichten der Bildsensoren insbesondere in derselben Ebene.

Bei einer Vorrichtung, wie sie hier beschrieben ist, liegen die lichtempfindlichen Schichten der Bildsensoren insbesondere in derselben Ebene, wobei die erste bildseitige Hauptebene des ersten Objektivs und die zweite bildseitige Hauptebene des zweiten Objektivs parallel zu einander und voneinander beabstandet sind oder die Schnittlinie der bildseitigen Hauptebenen der Objektive nicht die Hauptblickachse der Vorrichtung schneidet.

Zwei Ebenen, die nicht parallel sind, schneiden sich in einer geraden Schnittlinie. Wenn die Schnittlinie der Hauptebenen der Objektive die Hauptblickachse der Vorrichtung nicht schneidet, liegt sie seitlich der Hauptblickrichtung. Dies ist gleichbedeutend damit, dass die Hauptebenen nicht spiegelsymmetrisch angeordnet sind.

Mit einer parallelen Anordnung der Hauptebenen der Objektive ist eine innerhalb der Fertigungstoleranzen parallele Anordnung der Hauptebenen gemeint. Mit einer von einander beabstandeten Anordnung der Hauptebenen der Objektive ist ein Abstand der Hauptebenen der Objektive gemeint, der größer ist als die Fertigungstoleranz der Positionen der Hauptebenen der Objektive.

Eine parallele und voneinander beabstandete Anordnung der bildseitigen Hauptebenen der Objektive entspricht mathematisch einer Anordnung der Schnittlinie der Hauptebenen im Unendlichen. Die Schnittlinie der bildseitigen Hauptebenen der Objektive schneidet die Hauptblickachse der Vorrichtung nicht, wenn ihr Abstand von der Hauptblickachse außerhalb der Fertigungstoleranzen liegt.

Ein Abstand der Schnittlinie der bildseitigen Hauptebenen der Objektive von der Hauptblickachse charakterisiert - auch im Falle nicht paralleler optischer Achsen der Objektive - eine asymmetrische Anordnung der bildseitigen Hauptebenen der Objektive der Vorrichtung. Diese asymmetrische Anordnung der bildseitigen Hauptebenen der Objektive ermöglicht ein Auseinanderfallen der ersten Fläche, in der Punkte liegen, die scharf auf die erste lichtempfindliche Schicht des ersten Bildsensors abgebildet werden, und der zweiten Fläche, in der Punkte liegen, die scharf auf die zweite lichtempfindliche Schicht des zweiten Bildsensors abgebildet werden.

Bei einer Vorrichtung, wie sie hier beschrieben ist, liegen insbesondere die erste lichtempfindliche Schicht des ersten Bildsensors in einer ersten Ebene und die zweite lichtempfindliche Schicht des zweiten Bildsensors in einer zweiten Ebene, wobei die erste Ebene und die zweite Ebene parallel und voneinander beabstandet sind.

Bei einer Vorrichtung, wie sie hier beschrieben ist, liegen die lichtempfindlichen Schichten der Bildsensoren insbesondere in zwei verschiedenen Ebenen, die parallel und voneinander beabstandet sind oder deren Schnittlinie nicht die Hauptblickachse der Vorrichtung schneidet.

Eine Anordnung der lichtempfindlichen Schichten der Bildsensoren in zwei verschiedenen Ebenen kann mit einer Anordnung der bildseitigen Hauptebenen der Objektive in einer Ebene oder in zwei voneinander verschiedenen Ebenen kombiniert werden, um ein Auseinanderfallen der ersten Fläche, die scharf auf die erste lichtempfindliche Schicht des ersten Bildsensors abgebildet wird, und der zweiten Fläche, die scharf auf die zweite lichtempfindliche Schicht des zweiten Bildsensors abgebildet wird, zu bewirken.

Bei einer Vorrichtung, wie sie hier beschrieben ist, sind die erste bildseitige Hauptebene des ersten Objektivs und die zweite bildseitige Hauptebene des zweiten Objektivs insbesondere identisch, wobei die Bildsensoren so angeordnet sind, dass die lichtempfindlichen Schichten der Bildsensoren in zwei parallelen und von einander beabstandeten Ebenen liegen.

Eine weitere erfindungsgemäße Vorrichtung zum Erfassen eines Stereobilds umfasst einen ersten Bildsensor mit einer ersten lichtempfindlichen Schicht zum Erfassen eines ersten Bilds, einen zweiten Bildsensor mit einer zweiten lichtempfindlichen Schicht zum Erfassen eines zweiten Bilds, ein erstes Objektiv mit einer ersten bildseitigen Hauptebene, das Punkte, die in einer ersten Fläche liegen, scharf auf die erste lichtempfindliche Schicht des ersten Bildsensors abbildet, und ein zweites Objektiv mit einer zweiten bildseitigen Hauptebene, das Punkte, die in einer zweiten Fläche liegen, scharf auf die zweite lichtempfindliche Schicht des zweiten Bildsensors abbildet, wobei das erste Objektiv und das zweite Objektiv einander gleichen, wobei der Abstand des ersten Objektivs von dem ersten Bildsensor dem Abstand des zweiten Objektivs von dem zweiten Bildsensor gleicht, und wobei das erste Objektiv und das zweite Objektiv in Richtung der Hauptblickachse der Vorrichtung versetzt angeordnet sind, so dass die erste Fläche und die zweite Fläche beabstandet oder in Richtung parallel zur Hauptblickachse der Vorrichtung versetzt sind.

Eine nicht erfindungsgemäße Vorrichtung zum Erfassen eines Stereobilds umfasst einen ersten Bildsensor mit einer ersten lichtempfindlichen Schicht zum Erfassen eines ersten Bilds, einen zweiten Bildsensor mit einer zweiten lichtempfindlichen Schicht zum Erfassen eines zweiten Bilds, ein erstes Objektiv mit einer ersten bildseitigen Hauptebene, das Punkte, die in einer ersten Fläche liegen, scharf auf die erste lichtempfindliche Schicht des ersten Bildsensors abbildet, und ein zweites Objektiv mit einer zweiten bildseitigen Hauptebene, das Punkte, die in einer zweiten Fläche liegen, scharf auf die zweite lichtempfindliche Schicht des zweiten Bildsensors abbildet, wobei die Anordnung des ersten Bildsensors und des ersten Objektivs der Anordnung des zweiten Bildsensors und des zweiten Objektivs gleicht, und wobei die Anordnung des ersten Bildsensors und des ersten Objektivs gegenüber der Anordnung des zweiten Bildsensors und des zweiten Objektivs in Richtung der Hauptblickachse der Vorrichtung versetzt ist, so dass die erste Fläche und die zweite Fläche beabstandet oder in Richtung parallel zur Hauptblickachse der Vorrichtung versetzt sind. Die Anordnung des ersten Bildsensors und des ersten Objektivs gleicht der Anordnung des zweiten Bildsensors und des zweiten Objektivs in allen geometrischen Eigenschaften und insbesondere auch in allen optischen Eigenschaften der Objektive. Optische oder geometrische Eigenschaften gleichen einander, wenn sie sich nicht oder nur im Umfang von Serienstreuung, Toleranzen und durch Justage erzeugten Ungleichheiten unterscheiden.

Von dem Versatz in der Hauptblickrichtung abgesehen sind die Anordnung des ersten Bildsensors und des ersten Objektivs und die Anordnung des zweiten Bildsensors und des zweiten Objektivs insbesondere spiegelsymmetrisch zu einer Symmetriebene, die die Hauptblickachse der Vorrichtung enthält.

Bei einer Vorrichtung, wie sie hier beschrieben ist, weisen insbesondere das erste Objektiv und der erste Bildsensor einen ersten Schärfentiefenbereich und das zweite Objektiv und der zweite Bildsensor einen zweiten Schärfentiefenbereich auf, wobei der erste Schärfentiefenbereich und der zweite Schärfentiefenbereich überlappen.

Der erste Schärfentiefenbereich des ersten Objektivs und des ersten Bildsensors ist insbesondere der minimale Bereich im Gegenstandsraum, innerhalb dessen alle Punkte liegen, die durch das erste Objektiv auf Bereiche (auch als Zerstreuungs- oder Unschärfekreise bezeichnet) innerhalb der ersten lichtempfindlichen Fläche des ersten Bildsensors abgebildet werden, die nicht größer als eine lichtempfindliche Zelle des ersten Bildsensors sind.

Der zweite Schärfentiefenbereich des zweiten Objektivs und des zweiten Bildsensors ist insbesondere der minimale Bereich, innerhalb dessen alle Punkte liegen, die durch das zweite Objektiv auf Bereiche innerhalb der zweiten lichtempfindlichen Fläche des zweiten Bildsensors abgebildet werden, die nicht größer als eine lichtempfindliche Zelle des zweiten Bildsensors sind.

Eine lichtempfindliche Zelle oder Bilderfassungszelle eines Bildsensors wird oft auch als Pixel bezeichnet. Wenn weniger Pixel bzw. Bildpunkte dargestellt als erfasst werden, kann die Schärfentiefe entsprechend größer sein. Wenn beispielsweise bei der Verarbeitung oder Aufbereitung des von einem Bildsensor erfassten Bilds jeweils die Signale einer Gruppe von 2x2 lichtempfindlichen Zellen zusammengefasst werden, ist ein Schärfentiefenbereich der Bereich, innerhalb dessen alle Punkte liegen, die durch das zugeordnete Objektiv auf einen Bereich der lichtempfindlichen Fläche des zugeordneten Bildsensors abgebildet werden, dessen lineare Abmessungen nicht größer als die linearen Abmessungen einer Gruppe von 2x2 lichtempfindlichen Zellen des zugeordneten Bildsensors ist.

Alternativ ist der Schärfentiefenbereich einer Anordnung aus einem Objektiv und einem Bildsensor der minimale Bereich, innerhalb dessen alle Punkte liegen, die durch das Objektiv auf einen Zerstreuungs- oder Unschärfekreis abgebildet werden, dessen Durchmesser nicht mehr als einen vorbestimmten Bruchteil (beispielsweise 1/1500) der Länge der Diagonale der lichtempfindlichen Schicht des zugeordneten Bildsensors beträgt.

Ein Überlapp der Schärfentiefenbereiche ermöglicht, dass alle Objekte innerhalb eines vergrößerten Bereichs von mindestens einem Auge des Betrachters scharf erfasst und damit vom Betrachter als scharf abgebildet wahrgenommen werden können. Dieser vergrößerte Bereich umfasst insbesondere eine Vereinigung beider Schärfentiefenbereiche.

Bei einer Vorrichtung, wie sie hier beschrieben ist, liegen insbesondere zumindest entweder die erste Fläche innerhalb des zweiten Schärfentiefenbereichs oder die zweite Fläche innerhalb des ersten Schärfentiefenbereichs.

Bei einer Vorrichtung, wie sie hier beschrieben ist, liegen insbesondere die erste Fläche innerhalb des zweiten Schärfentiefenbereichs und die zweite Fläche innerhalb des ersten Schärfentiefenbereichs.

Insbesondere liegen die erste Fläche nahe an einem Rand des zweiten Schärfentiefenbereichs und die zweite Fläche nahe an einem Rand des ersten Schärfentiefenbereichs. Eine Fläche liegt nahe an einem Rand eines Schärfentiefenbereichs, wenn sie den Schärfentiefenbereich in einem Verhältnis von mindestens 3:1 oder mindestens 4:1 oder mindestens 9:1 teilt.

Ein Überlapp der Schärfentiefenbereiche derart, dass die erste Fläche innerhalb des zweiten Schärfentiefenbereichs und/oder die zweite Fläche innerhalb des ersten Schärfentiefenbereichs liegt, kann einen großen Überlappbereich der beiden Schärfentiefenbereiche, innerhalb dessen beide Augen eines Betrachters ein scharfes Bild erfassen können, und gleichzeitig große angrenzende Bereiche, innerhalb derer ein Auge eines Betrachters ein scharfes Bild erfassen kann, erzeugen. Dies kann eine ausgeprägte räumliche Wahrnehmung fördern.

Bei einer Vorrichtung, wie sie hier beschrieben ist, überlappen die Schärfentiefenbereiche insbesondere zu mindestens der Hälfte der Tiefe des ersten Schärfentiefenbereichs und zu mindestens der Hälfte der Tiefe des zweiten Schärfentiefenbereichs und zu höchstens zwei Dritteln oder drei Vierteln der Tiefe des ersten Schärfentiefenbereichs und zu höchstens zwei Dritteln oder drei Vierteln der Tiefe des zweiten Schärfentiefenbereichs.

Alternativ können die Schärfentiefenbereiche beispielsweise zu mindestens der Hälfte oder zwei Dritteln der Tiefe des ersten Schärfentiefenbereichs und zu mindestens der Hälfte oder zwei Dritteln der Tiefe des zweiten Schärfentiefenbereichs und zu höchstens drei Vierteln oder vier Fünfteln oder sieben Achteln der Tiefe des ersten Schärfentiefenbereichs und zu höchstens drei Vierteln oder vier Fünfteln oder sieben Achteln der Tiefe des zweiten Schärfentiefenbereichs überlappen.

Eine Vorrichtung, wie sie hier beschrieben ist, umfasst insbesondere ferner eine Fokussiereinrichtung zum synchronen Bewegen des ersten Objektivs und des zweiten Objektivs zum gleichzeitigen Bewegen der ersten Fläche und der zweiten Fläche.

Eine Bewegung eines Objektivs relativ zu dem zugeordneten Bildsensor hat eine Bewegung derjenigen Fläche, die durch das Objektiv scharf auf den Bildsensor abgebildet wird, zur Folge. Eine synchrone Bewegung des ersten Objektivs und des zweiten Objektivs durch die Fokussiereinrichtung hat eine gleichzeitige Bewegung der ersten Fläche, die durch das erste Objektiv scharf auf den ersten Bildsensor abgebildet wird, und der zweiten Fläche, die durch das zweite Objektiv scharf auf den zweiten Bildsensor abgebildet wird, zur Folge.

Eine Vorrichtung, wie sie hier beschrieben ist, umfasst insbesondere ferner einen Schlitten, an dem das erste Objektiv starr oder in einer Richtung, die nicht parallel zu der optischen Achse des Objektivs ist, bewegbar befestigt ist, und an dem das zweite Objektiv starr oder bewegbar befestigt ist.

Die erfindungsgemäße Vorrichtung umfasst ferner einen Schlitten, an dem das erste Objektiv starr oder in einer Richtung, die nicht parallel zu der optischen Achse des Objektivs ist, bewegbar befestigt ist, und an dem das zweite Objektiv starr oder bewegbar befestigt ist, und eine Schlitten-Antriebseinrichtung zum Bewegen des Schlittens zum gleichzeitigen Bewegen der ersten Fläche und der zweiten Fläche.

Das erste Objektiv ist an dem Schlitten insbesondere starr oder in einer Richtung, die orthogonal zu der optischen Achse des Objektivs ist, bewegbar. Eine Bewegbarkeit des ersten Objektivs relativ zu dem Schlitten kann insbesondere eine Veränderung der Disparität eines mittels der Vorrichtung erfassten Stereobilds ermöglichen.

Der Schlitten ist relativ zu den Bildsensoren insbesondere in Richtung parallel zu mindestens einer der optischen Achsen der Objektive oder parallel zu der Hauptblickachse der Vorrichtung bewegbar, um die erste Fläche und die zweite Fläche gleichzeitig mittels einer einzigen Schlitten-Antriebseinrichtung zu bewegen. Ein Steuerungs- oder Regelungsaufwand zur Abstimmung zweier an sich mechanisch unabhängiger Bewegungen aufeinander entfällt damit.

Die Vorrichtung kann ferner einen Positionssensor zur Erfassung der Position des Schlittens umfassen. Alternativ kann die Schlitten-Antriebseinrichtung zur Erfassung einer Position des Schlittens vorgesehen und ausgebildet sein.

Eine Vorrichtung, wie sie hier beschrieben ist, umfasst insbesondere ferner eine Objektiv-Antriebseinrichtung zum Bewegen des zweiten Objektivs oder eines Teils des zweiten Objektivs relativ zu dem Schlitten und zu dem ersten Objektiv und zum Bewegen der zweiten Fläche relativ zu der ersten Fläche.

Die Antriebseinrichtung ist insbesondere zum Bewegen des zweiten Objektivs oder eines Teils des zweiten Objektivs in einer Richtung parallel zu der optischen Achse des zweiten Objektivs vorgesehen und ausgebildet.

Eine Bewegung eines Teils des zweiten Objektivs (insbesondere einer oder mehrerer Linsen oder Linsengruppen) erfolgt so, dass dabei im Wesentlichen nur die zweite Fläche, die scharf auf die zweite lichtempfindliche Schicht des zweiten Bildsensors abgebildet wird, bewegt wird (d. h. ihre Abstände von dem Objektiv und dem Bildsensor verändert), jedoch im Wesentlichen nicht der Bildausschnitt verändert wird. Das zweite Objektiv ist also insbesondere kein pankratisches System (oft auch als Zoom- oder Vario-Objektiv bezeichnet), mittels dessen ein Bildausschnitt mehr als nur unwesentlich verändert werden kann. Die Objektiv-Antriebseinrichtung ist insbesondere vorgesehen und ausgebildet, um das zweite Objektiv oder einen Teil des zweiten Objektivs in Richtung parallel zu der optischen Achse des zweiten Objektivs zu bewegen.

Die Vorrichtung kann ferner einen Positionssensor zur Erfassung der Position des zweiten Objektivs oder eines Teils des zweiten Objektivs relativ zu dem Schlitten und/oder zu dem ersten Objektiv umfassen. Alternativ kann die Objektiv-Antriebseinrichtung zur Erfassung einer Position des zweiten Objektivs oder eines Teils des zweiten Objektivs relativ zu dem Schlitten und/oder zu dem ersten Objektiv vorgesehen und ausgebildet sein.

Die Objektiv-Antriebseinrichtung kann eine Vergrößerung oder Verkleinerung oder allgemeiner formuliert eine Veränderung des Überlappbereichs zwischen dem ersten Schärfentiefenbereich und dem zweiten Schärfentiefenbereich ermöglichen. Die Objektiv-Antriebseinrichtung kann damit eine Anpassung der Vorrichtung an die Entfernung zu dem betrachteten Objekt (insbesondere an eine besonders große oder an eine besonders kleine Entfernung), an eine bestimmte Anwendung, eine bestimmte Situation oder an Sehgewohnheiten oder Vorlieben eines Benutzers ermöglichen. Ohne Steuerung der Objektiv-Antriebseinrichtung werden durch Bewegung des Schlittens beide Schärfentiefenbereiche gleichzeitig bewegt.

Verschiedene Funktionen, nämlich einerseits die gleichzeitige Bewegung der ersten Fläche und der zweiten Fläche und damit auch die gleichzeitige Bewegung beider Schärfentiefenbereiche und andererseits die Veränderung des Überlapps der Schärfentiefenbereiche, werden damit durch verschiedene Antriebseinrichtungen realisiert. Dies kann eine einfache und benutzerfreundliche Steuerung ermöglichen.

Eine Vorrichtung, wie sie hier beschrieben ist, umfasst insbesondere ferner eine Fokussiereinrichtung zum synchronen Bewegen des ersten Bildsensors und des zweiten Bildsensors zum gleichzeitigen Bewegen der ersten Fläche und der zweiten Fläche.

Die Fokussiereinrichtung ist insbesondere vorgesehen und ausgebildet, um den ersten Bildsensor parallel zu der optischen Achse des ersten Objektivs und/oder parallel zu der Flächennormalen der ersten lichtempfindlichen Schicht des ersten Bildsensors zu bewegen. Die Fokussiereinrichtung ist insbesondere ferner vorgesehen und ausgebildet, um den zweiten Bildsensor parallel zu der optischen Achse des zweiten Objektivs und/oder parallel zu der Flächennormalen der zweiten lichtempfindlichen Schicht des zweiten Bildsensors zu bewegen. Alternativ oder zusätzlich kann die Fokussiereinrichtung ausgebildet sein, um beide Bildsensoren parallel zu der Hauptblickachse der Vorrichtung zu bewegen.

Eine Vorrichtung, wie sie hier beschrieben ist, umfasst insbesondere ferner einen Schlitten, an dem der erste Bildsensor starr oder in einer Richtung, die parallel zu der lichtempfindlichen Schicht des ersten Bildsensors ist, bewegbar befestigt ist, und an dem der zweite Bildsensor starr oder bewegbar befestigt ist, und eine Schlitten-Antriebseinrichtung zum Bewegen des Schlittens zum gleichzeitigen Bewegen der ersten Fläche und der zweiten Fläche.

Eine Bewegbarkeit des ersten Bildsensors relativ zu dem Schlitten kann insbesondere eine Veränderung der Disparität eines mittels der Vorrichtung erfassten Stereobilds ermöglichen.

Der Schlitten ist insbesondere derart spiel- und reibungsarm geführt und die Schlitten-Antriebseinrichtung ist insbesondere vorgesehen und ausgebildet, um den Schlitten in einer Richtung parallel zu mindestens einer optischen Achse eines Objektivs oder parallel zu der Hauptblickachse der Vorrichtung zu bewegen.

Die Vorrichtung kann ferner einen Positionssensor zur Erfassung der Position des Schlittens umfassen. Alternativ kann die Schlitten-Antriebseinrichtung zur Erfassung einer Position des Schlittens vorgesehen und ausgebildet sein.

Wenn die Bildsensoren durch eine Fokussiereinrichtung oder durch eine Schlitten-Antriebseinrichtung bewegbar sind, können die Objektive starr bzw. unbewegbar in der Vorrichtung angeordnet oder ebenfalls relativ zu der Vorrichtung bewegbar sein.

Eine Vorrichtung, wie sie hier beschrieben ist, umfasst insbesondere ferner eine Bildsensor-Antriebseinrichtung zum Bewegen des zweiten Bildsensors relativ zu dem ersten Bildsensor und zum Bewegen der zweiten Fläche relativ zu der ersten Fläche.

Eine Vorrichtung, wie sie hier beschrieben ist, umfasst insbesondere ferner eine Bildsensor-Antriebseinrichtung zum Bewegen des zweiten Bildsensors relativ zu dem Schlitten und zu dem ersten Bildsensor und zum Bewegen der zweiten Fläche relativ zu der ersten Fläche.

Die Bildsensor-Antriebseinrichtung ist insbesondere zum Bewegen des zweiten Bildsensors relativ zu dem ersten Bildsensor und/oder relativ zu dem Schlitten in Richtung parallel zu der optischen Achse des zweiten Objektivs und/oder parallel zu der Flächennormalen der lichtempfindlichen Schicht des zweiten Bildsensors und/oder parallel zu der Hauptblickachse der Vorrichtung vorgesehen und ausgebildet.

Die Vorrichtung kann ferner einen Positionssensor zur Erfassung der Position des zweiten Bildsensors relativ zu dem Schlitten und/oder zu dem ersten Bildsensor umfassen. Alternativ kann die Bildsensor-Antriebseinrichtung zur Erfassung einer Position des zweiten Bildsensors relativ zu dem Schlitten und/oder zu dem ersten Bildsensor vorgesehen und ausgebildet sein.

Ein erfindungsgemäßes Verfahren zum Justieren einer Vorrichtung zum Erfassen eines Stereobildsignals bei Herstellung oder Wartung der Vorrichtung umfasst einen Schritt des Justierens zumindest entweder eines ersten Objektivs oder eines ersten Bildsensors der Vorrichtung derart, dass das erste Objektiv ein scharfes Bild eines ersten Objekts an dem ersten Bildsensor erzeugt, und einen Schritt des Justierens zumindest entweder eines zweiten Objektivs oder eines zweiten Bildsensors der Vorrichtung derart, dass das zweite Objektiv ein scharfes Bild eines zweiten Objekts an dem zweiten Bildsensor erzeugt, wobei das erste Objekt in einer ersten vorbestimmten Ebene liegt, wobei das zweite Objekt in einer zweiten vorbestimmten Ebene liegt, wobei die erste Ebene und die zweite Ebene jeweils orthogonal zu einer Hauptblickachse der Vorrichtung sind, und wobei die erste vorbestimmte Ebene und die zweite vorbestimmte Ebene parallel zueinander und voneinander beabstandet sind.

Das Verfahren ist insbesondere mit einer Vorrichtung, wie sie hier beschrieben ist, ausführbar. Mittels des Verfahrens ist insbesondere eine Vorrichtung, wie sie hier beschrieben ist, justierbar.

Das erfindungsgemäße Verfahren umfasst ferner einen Schritt des Anordnens einer Justierlehre mit dem ersten Objekt und mit dem zweiten Objekt an einer vorbestimmten Position relativ zu der Vorrichtung derart, dass das erste Objekt in der ersten vorbestimmten Ebene und das zweite Objekt in der zweiten vorbestimmten Ebene angeordnet sind.

Der Schritt des Anordnens einer Justierlehre wird vor dem Schritt des Justierens zumindest entweder des ersten Objektivs oder des ersten Bildsensors und vor dem Schritt des Justierens zumindest entweder des zweiten Objektivs oder des zweiten Bildsensors ausgeführt.

Ein Verfahren, wie es hier beschrieben ist, umfasst insbesondere ferner einen Schritt des Fixierens zumindest entweder der Bildsensoren relativ zueinander oder der Objektive relativ zueinander.

Der Schritt des Fixierens wird nach den Schritten des Justierens ausgeführt.

Der Schritt des Justierens zumindest entweder des ersten Objektivs oder des ersten Bildsensors wird insbesondere durch Justieren eines Schlittens, an dem entweder das erste Objektiv oder der erste Bildsensor starr befestigt ist, ausgeführt. Der Schritt des Justierens zumindest entweder des zweiten Objektivs oder des zweiten Bildsensors wird insbesondere nach dem Schritt des Justierens zumindest entweder des ersten Objektivs oder des ersten Bildsensors ausgeführt. Der Schritt des Justierens zumindest entweder des zweiten Objektivs oder des zweiten Bildsensors wird insbesondere durch Justieren des zweiten Objektivs relativ zu dem ersten Objektiv und/oder zu einem Schlitten, an dem das erste Objektiv starr befestigt ist oder durch Justieren des zweiten Bildsensors relativ zu dem ersten Bildsensor und/oder relativ zu einem Schlitten, an dem der erste Bildsensor starr befestigt ist, ausgeführt.

Nach dem Justieren der Vorrichtung mit dem beschriebenen Verfahren kann die Vorrichtung verwendet werden. Bei der vorgesehenen Verwendung der Vorrichtung können beide Objektive synchron (insbesondere zusammen mit einem Schlitten, an dem die Objektive befestigt sind) bewegt werden. Alternativ können bei der vorgesehenen Verwendung der Vorrichtung beide Bildsensoren synchron (insbesondere zusammen mit einem Schlitten, an dem die Bildsensoren befestigt sind) bewegt werden. Die synchrone Bewegung kann manuell oder mittels eines Motors, der manuell oder durch eine Steuerungseinrichtung gesteuert wird, erfolgen. Mit einer synchronen Bewegung beider Objektive oder beider Bildsensoren können die erste Fläche, die durch das erste Objektiv scharf in die lichtempfindliche Schicht des ersten Bildsensors abgebildet wird, und die zweite Fläche, die durch das zweite Objektiv scharf in die lichtempfindliche Schicht des zweiten Bildsensors abgebildet wird, gleichzeitig bewegt werden.

Eine Steuerungseinrichtung zur Steuerung einer motorischen synchronen Bewegung beider Bildsensoren oder beider Objektive kann - beispielsweise auf der Grundlage der Bestimmung eines Kantenkontrasts - eine automatische Fokussierung ermöglichen.

### Kurzbeschreibung der Figuren

Nachfolgend werden Ausführungsformen anhand der beigefügten Figuren näher erläutert. Es zeigen:
- Figur 1: eine schematische Darstellung einer Vorrichtung zum Erfassen eines Stereobilds;
- Figur 2: eine schematische Darstellung optischer Abbildungen in einer Vorrichtung zum Erfassen eines Stereobilds;
- Figur 3: eine schematische Darstellung einer weiteren Vorrichtung zum Erfassen eines Stereobilds;
- Figur 4: eine schematische Darstellung einer weiteren Vorrichtung zum Erfassen eines Stereobilds;
- Figur 5: eine schematische Darstellung optischer Abbildungen in einer weiteren Vorrichtung zum Erfassen eines Stereobilds;
- Figur 6: eine schematische Darstellung optischer Abbildungen in einer weiteren Vorrichtung zum Erfassen eines Stereobilds;
- Figur 7: eine schematische Darstellung optischer Abbildungen in einer weiteren Vorrichtung zum Erfassen eines Stereobilds;
- Figur 8: eine schematische Darstellung einer weiteren Vorrichtung zum Erfassen eines Stereobilds;
- Figur 9: ein schematisches Flussdiagramm eines Verfahrens zum Justieren einer Vorrichtung zum Erfassen eines Stereobilds.

### Beschreibung der Ausführungsformen

Figur 1 zeigt eine schematische Darstellung einer erfindungsgemäßen Vorrichtung 10 zum Erfassen eines Stereobilds. Einige Teile der Vorrichtung 10 sind in einem Schnitt entlang einer Schnittebene dargestellt. Insofern weist die Darstellung in Figur 1 teilweise den Charakter einer Schnittdarstellung auf. Einige Schnittflächen sind mit Schraffuren versehen. Andere Schnittflächen, beispielsweise von Bauteilen aus optisch transparenten Materialien, sind ohne Schraffur dargestellt.

Die Vorrichtung 10 umfasst ein Gehäuse 12 mit einer Öffnung, die durch ein Fensterbauteil 14 verschlossen ist. Das Fensterbauteil 14 ist gebildet aus einem Material, das für Licht zumindest innerhalb des für das gesunde menschliche Auge sichtbaren Wellenlängenbereichs transparent ist. Das Gehäuse 12 mit dem Fensterbauteil 14 kann fluiddicht, optional hermetisch dicht sein. Eine äußere (in Figur 1: untere) Oberfläche des Fensterbauteils 14 bildet eine Lichteintrittsfläche 16, durch die von einem zu beobachtenden Objekt ausgehendes Licht in das Gehäuse 12 und die Vorrichtung 10 eintreten kann.

Die Vorrichtung 10 weist eine Hauptblickrichtung und Hauptblickachse 18 auf. Die Hauptblickachse 18 der Vorrichtung 10 liegt in der Mitte des Bereichs, innerhalb dessen alle Gegenstände liegen können, von denen die Vorrichtung 10 ein Stereobild erfassen kann.

Die Vorrichtung 10 ist mit einer Wiedergabevorrichtung 20 zum Wiedergeben eines von der Vorrichtung 10 erfassten Stereobilds gekoppelt. Die Wiedergabevorrichtung 20 umfasst beispielsweise einen Projektor und eine Projektionsfläche oder einen oder mehrere Bildschirme zur gleichzeitigen oder schnell alternierenden Darstellung der für das linke und für das rechte Auge eines Betrachters vorgesehenen Bilder eines durch die Vorrichtung 10 erfassten Stereobilds. Die Wiedergabevorrichtung 20 kann Bestandteil der Vorrichtung 10 sein oder mit der Vorrichtung 10 zu einem System kombiniert werden.

Die Vorrichtung 10 umfasst einen ersten Bildsensor 30 zum Erfassen eines für ein erstes (beispielsweise linkes) Auge eines Betrachters vorgesehenen Bilds und einen zweiten Bildsensor 40 zur Erfassung eines für ein zweites (beispielsweise rechtes) Auge des Betrachters vorgesehenen Bilds. Die Bildsensoren 30, 40 sind beispielsweise CCD- oder CMOS-Sensoren. Die Bildsensoren 30, 40 sind ausgebildet, um (insbesondere elektrische) analoge oder digitale Bildsignale, die die von den Bildsensoren 30, 40 erfassten Bilder repräsentieren, zu erzeugen. Von den Bildsensoren 30, 40 erzeugte Bildsignale können durch in Figur 1 nicht dargestellte Einrichtungen verstärkt, aufbereitet und/oder bearbeitet und danach gespeichert und/oder durch die Wiedergabevorrichtung 20 oder auf andere Weise wiedergegeben werden.

Jeder Bildsensor 30, 40 weist eine Vielzahl von Pixeln bzw. Bilderfassungszellen bzw. lichtempfindlichen Zellen 31, 41 auf. Die lichtempfindlichen Zellen 31, 41 sind in lichtempfindlichen Schichten 32, 42 angeordnet. Bei dem dargestellten Beispiel sind die lichtempfindlichen Schichten 32, 42 der Bildsensoren 30, 40 jeweils eben. Da die lichtempfindlichen Schichten 32, 42 der Bildsensoren 30, 40 jeweils sehr dünn sind, wird nachfolgend auf Ebenen 33, 43 Bezug genommen, in denen die lichtempfindlichen Schichten 32, 42 der Bildsensoren 30, 40 liegen. Eine präzisere Definition der Ebenen 33, 43 ist, dass diese die lichtempfindlichen Schichten 32, 42 der Bildsensoren 30, 40 mittig schneiden, so dass jeweils die Hälfte aller zur optoelektronischen Bildwandlung beitragenden Elementarereignisse vor und hinter der Ebene 33, 43 stattfindet. Ferner wird nachfolgend auf Flächennormalen 38, 48 der Ebenen 33, 43, in denen die lichtempfindlichen Schichten 32, 42 der Bildsensoren 30, 40 liegen, Bezug genommen.

In Figur 1 sind die Bildsensoren 30, 40 als separate und voneinander beabstandete Bauelemente angedeutet. Alternativ und abweichend von der Darstellung in Figur 1 können beide Bildsensoren 30, 40 in einem einzigen gemeinsamen Bauelement realisiert sein, beispielsweise als zwei verschiedene (und insbesondere räumliche beabstandete) Bereiche einer durchgehenden lichtempfindlichen Schicht eines Halbleiterbauteils.

Die Vorrichtung 10 umfasst ferner ein erstes Objektiv 50, das dem ersten Bildsensor 30 zugeordnet ist, und ein zweites Objektiv 60, das dem zweiten Bildsensor 40 zugeordnet ist. Jedes Objektiv 50, 60 weist eine bildseitige Hauptebene 52, 62, eine objektseitige Hauptebene 54, 64 und eine optische Achse 58, 68 auf. Als Hauptebenen werden die im Rahmen des Arbeitsmodells der paraxialen Optik zur Beschreibung der Brechung von Licht durch die Objektive 50, 60 verwendeten Hauptebenen angesehen. Jedes Objektiv 50, 60 kann eine oder mehrere Linsen mit gekrümmten lichtbrechenden Oberflächen und/oder inhomogenem Brechungsindex, reflektierende Flächen und/oder lichtbeugende oder andere optische Elemente aufweisen. In Figur 1 ist jedes Objektiv 50, 60 beispielhaft durch eine einzige Linse repräsentiert.

Bei dem in Figur 1 gezeigten Beispiel sind die Hauptebenen 52, 54, 62, 64 der Objektive 50, 60 parallel zu den Ebenen 33, 43, in denen die lichtempfindlichen Schichten 32, 42 der Bildsensoren 30, 40 liegen. Damit sind die optischen Achsen 58, 68 der Objektive 50, 60 gleichzeitig Flächennormale 38, 48 der Ebenen 33, 43, in denen die lichtempfindlichen Schichten 32, 42 der Bildsensoren 30, 40 liegen.

Das erste Objektiv 50 bildet - von Beugung am Rand des ersten Objektivs 50 und Abbildungsfehlern abgesehen - Punkte in einer ersten Fläche 70 scharf (von Abbildungsfehlern abgesehen: punktförmig) in die lichtempfindliche Schicht 32 des ersten Bildsensors 30 ab. Je größer der Abstand eines Punkts im Objektraum von der ersten Fläche 70 ist, desto größer ist der Bereich innerhalb der lichtempfindlichen Schicht 32 des ersten Bildsensors 30, in den von dem Punkt ausgehendes und durch das erste Objektiv 50 gebrochenes Licht fällt. Dieser Bereich wird auch als Zerstreuungs- oder Unschärfekreis bezeichnet. Ein erster Schärfentiefenbereich 73 der in Figur 1 gezeigten Anordnung aus dem ersten Bildsensor 30 und dem ersten Objektiv 50 ist der minimale Bereich des Objektraums, innerhalb dessen alle Punkte liegen, deren durch das erste Objektiv 50 erzeugte Bilder in der lichtempfindlichen Schicht 32 des ersten Bildsensors 30 nicht größer als eine lichtempfindliche Zelle 31 des ersten Bildsensors 30 sind. Der erste Schärfentiefenbereich 73 erstreckt sich von einer Ferngrenze bzw. einem distalen Rand 74 bis zu einer Nahgrenze bzw. einem proximalen Rand 75.

Bei dem dargestellten Beispiel sind die erste Fläche 70 und die Ränder 74, 75 des ersten Schärfentiefenbereichs 73 jeweils eben oder im Wesentlichen eben und parallel zu den Hauptebenen 52, 54 des ersten Objektivs 50 und zu der Ebene 33, in der die lichtempfindliche Schicht 32 des ersten Bildsensors 30 liegt. Die Tiefe t₁ des ersten Schärfentiefenbereichs 73 ist von der Größe der einzelnen lichtempfindlichen Zellen 31 des ersten Bildsensors 30 und von Eigenschaften (insbesondere der Größe der Aperturblende) des ersten Objektivs 50 abhängig.

Der Abstand der ersten Fläche 70 von der objektseitigen Hauptebene 54 des ersten Objektivs 50 wird als Gegenstandsweite bzw. Objektweite g₁ bezeichnet. Der Abstand der Ebene 33, in der die lichtempfindliche Schicht 32 des ersten Bildsensors 30 liegt, von der bildseitigen Hauptebene 52 des ersten Objektivs 50 wird als Bildweite b₁ bezeichnet.

Das zweite Objektiv 60 bildet - von Beugung am Rand des zweiten Objektivs 60 und Abbildungsfehlern abgesehen - Punkte in einer zweiten Fläche 80 scharf (von Abbildungsfehlern abgesehen: punktförmig) in die lichtempfindliche Schicht 42 des zweiten Bildsensors 40 ab. Je grö-ßer der Abstand eines Punkts im Objektraum von der zweiten Fläche 80 ist, desto größer ist der Bereich innerhalb der lichtempfindlichen Schicht 42 des zweiten Bildsensors 40, in den von dem Punkt ausgehendes und durch das zweite Objektiv 60 gebrochenes Licht fällt. Ein zweiter Schärfentiefenbereich 83 der in Figur 1 gezeigten Anordnung aus dem zweiten Bildsensor 40 und dem zweiten Objektiv 60 ist der minimale Bereich des Objektraums, innerhalb dessen alle Punkte liegen, deren durch das zweite Objektiv 60 erzeugte Bilder in der lichtempfindlichen Schicht 42 des zweiten Bildsensors 40 nicht größer als eine lichtempfindliche Zelle 41 des zweiten Bildsensors 40 sind. Der zweite Schärfentiefenbereich 83 erstreckt sich von einer Ferngrenze bzw. einem distalen Rand 84 bis zu einer Nahgrenze bzw. einem proximalen Rand 85.

Bei dem dargestellten Beispiel sind die zweite Fläche 80 und die Ränder 84, 85 des zweiten Schärfentiefenbereichs 83 jeweils eben oder im Wesentlichen eben und parallel zu den Hauptebenen 62, 64 des zweiten Objektivs 60 und zu der Ebene 43, in der die lichtempfindliche Schicht 42 des zweiten Bildsensors 40 liegt. Die Tiefe t₂ des zweiten Schärfentiefenbereichs 83 ist von der Größe der einzelnen lichtempfindlichen Zellen 41 des zweiten Bildsensors 40 und von Eigenschaften (insbesondere der Größe der Aperturblende) des zweiten Objektivs 60 abhängig.

Der Abstand der zweiten Fläche 80 von der objektseitigen Hauptebene 64 des zweiten Objektivs 60 wird als Gegenstandsweite bzw. Objektweite g₂ bezeichnet. Der Abstand der Ebene 43, in der die lichtempfindliche Schicht 42 des zweiten Bildsensors 40 liegt, von der bildseitigen Hauptebene 62 des zweiten Objektivs 60 wird als Bildweite b₂ bezeichnet.

Bei der in Figur 1 gezeigten Vorrichtung 10 weisen die Objektive 50, 60 innerhalb der Fertigungstoleranz gleiche optische Eigenschaften, insbesondere gleiche Brennweiten, auf, und die Bildweiten b₁ und b₂ unterscheiden sich. Entsprechend sind auch die Objektweiten g₁ und g₂ unterschiedlich. Die Ebenen 33, in der die lichtempfindliche Schicht 32 des ersten Bildsensors 30 liegt, ist mit der Ebene 43, in der die lichtempfindliche Schicht 42 des zweiten Bildsensors 40 liegt, identisch. Die erste Fläche 70, die durch das erste Objektiv 50 scharf in die lichtempfindliche Schicht 32 des ersten Bildsensors 30 abgebildet wird, ist von der zweiten Fläche 80, die durch das zweite Objektiv 60 scharf in die lichtempfindliche Schicht 42 des zweiten Bildsensors 40 abgebildet wird, beabstandet. Die Schärfentiefenbereiche 73, 83 überlappen einander derart, dass die erste Fläche 70, die durch das erste Objektiv 50 scharf in die lichtempfindliche Schicht 32 des ersten Bildsensors 30 abgebildet wird, innerhalb des zweiten Schärfentiefenbereichs 83 des zweiten Objektivs 60 und des zweiten Bildsensors 40 liegt, und dass die zweite Fläche 80, die durch das zweite Objektiv 60 scharf in die lichtempfindliche Schicht 42 des zweiten Bildsensors 40 abgebildet wird, innerhalb des ersten Schärfentiefenbereichs 73 des ersten Objektivs 50 und des ersten Bildsensors 30 liegt.

Das erste Objektiv 50 und das zweite Objektiv 60 sind nebeneinander in einem Schlitten 91 angeordnet und jeweils mit dem Schlitten 91 starr verbunden. Eine Antriebseinrichtung 92 ist vorgesehen und ausgebildet, um den Schlitten 91 in einer Richtung parallel zu der Hauptblickachse 18 der Vorrichtung 10 zu bewegen. Wenn die Antriebseinrichtung 92 den Schlitten 91 mit den Objektiven 50, 60 bewegt, werden die Bildweiten b₁, b₂ und entsprechend die Objektweiten g₁, g₂ gleichzeitig verändert. Durch die starre Anordnung der Objektive 50, 60 in dem Schlitten 91 bleibt dabei die Differenz der Bildweiten b₁, b₂ konstant.

Durch den Überlapp des ersten Schärfentiefenbereichs 73 der Anordnung aus dem ersten Objektiv 50 und dem ersten Bildsensor 30 und des zweiten Schärfentiefenbereichs 83 der Anordnung aus dem zweiten Objektiv 60 und dem zweiten Bildsensor 40 entsteht ein vergrößerter Gesamtbereich, innerhalb dessen mindestens eines der beiden erfassten Bilder scharf ist. An einen Kernbereich, der der Schnittmenge beider Schärfentiefenbereiche 73, 83 entspricht, und in dem beide Bilder scharf sind, schließen sich in beide Richtungen Bereiche an, in denen eines der beiden erfassten Bilder scharf ist. Der Bereich, innerhalb dessen Objekte von einem menschlichen Betrachter als scharf abgebildet wahrgenommen werden, und der Bereich, innerhalb dessen ein räumlicher Eindruck entsteht, sind deshalb größer als im Falle identischer Flächen 73, 83.

Figur 2 zeigt eine schematische Darstellung lediglich der an der optischen Abbildung von Objekten unmittelbar beteiligten Bauteile der Vorrichtung 10 aus Figur 1. Im Übrigen ähnelt die Darstellung in Figur 2 der Darstellung in Figur 1.

In Figur 2 ist eine Situation am Ende eines Justierverfahrens zum Justieren beider Strahlengänge gezeigt. Dazu ist eine Justierlehre 93 an einer vorbestimmten Position relativ zu der Vorrichtung 10 angeordnet. Die Justierlehre umfasst eine erste Fläche 94 und eine zweite Fläche 95. Die Flächen 94, 95 an der Justierlehre 93 erstrecken sich entlang zweier paralleler und voneinander beabstandeter Ebenen. An den Flächen 94, 95 an der Justierlehre 93 sind jeweils kontrastreiche Strukturen vorgesehen.

Bei der vorgesehenen und in Figur 2 dargestellten Anordnung der Justierlehre 93 sind die Ebenen, entlang derer sich die Flächen 94, 95 der Justierlehre 93 erstrecken, orthogonal zu der Hauptblickachse 18 der Vorrichtung 10. Bei der vorgesehenen und in Figur 2 gezeigten Anordnung der Justierlehre 93 schneidet die optische Achse 58 des ersten Objektivs 50 die erste Fläche 94 an der Justierlehre 93, und die optische Achse 68 des zweiten Objektivs 60 schneidet die zweite Fläche 95 an der Justierlehre 93.

Nach dem Anordnen der Justierlehre 93 an der vorgesehenen und in Figur 2 gezeigten Position wird das erste Objektiv 50 (insbesondere durch Bewegen des Schlittens 91 - vgl. Figur 1) so positioniert, dass das erste Objektiv 50 die erste Fläche 94 an der Justierlehre 93 (von Abbildungsfehlern abgesehen) scharf in die lichtempfindliche Schicht 32 des ersten Bildsensors 30 abbildet, dass also die erste Fläche 70 (vgl. Beschreibung der Figur 1) mit der ersten Fläche 94 an der Justierlehre zusammenfällt. Dies geschieht insbesondere durch Verschiebung des Schlittens 91 (vgl. Figur 1), an dem das erste Objektiv 50 starr befestigt ist.

Danach wird ohne eine weitere Bewegung des Schlittens 91 und des ersten Objektivs 50 das zweite Objektiv 60 so justiert, insbesondere so positioniert, dass das zweite Objektiv 60 die zweite Fläche 95 an der Justierlehre 93 scharf in die lichtempfindliche Schicht 42 des zweiten Bildsensors 40 abbildet, dass also die zweite Fläche 80 (vgl. Beschreibung der Figur 1) mit der zweiten Fläche 95 an der Justierlehre zusammenfällt. Dies geschieht insbesondere durch Verschiebung des zweiten Objektivs 60, das zu diesem Zeitpunkt noch nicht starr an dem Schlitten 91 befestigt ist, relativ zu dem Schlitten 91 (vgl. Figur 1).

Wenn gleichzeitig das erste Objektiv 50 die erste Fläche 94 an der Justierlehre 93 scharf in die lichtempfindliche Schicht 32 des ersten Bildsensors 30 abbildet und das zweite Objektiv 60 die zweite Fläche 95 an der Justierlehre 93 scharf in die lichtempfindliche Schicht 42 des zweiten Bildsensors 40 abbildet, wird das zweite Objektiv 60 relativ zu dem ersten Objektiv 50 fixiert. Dies geschieht insbesondere, indem das zweite Objektiv 60 - beispielsweise mittels eines Klebstoffs oder einer Klemmung - mit dem Schlitten 91 (vgl. Figur 1) starr verbunden wird.

Figur 3 zeigt eine schematische Darstellung einer weiteren erfindungsgemäßen Vorrichtung 10 zum Erfassen eines Stereobilds, die in einigen Merkmalen, Eigenschaften und Funktionen der anhand der Figuren 1 und 2 dargestellten Vorrichtung ähnelt. Die Art der Darstellung in Figur 3 entspricht derjenigen in Figur 1. Nachfolgend sind insbesondere Merkmale, Eigenschaften und Funktionen der in Figur 3 gezeigten Vorrichtung 10 beschrieben, in denen diese sich von der anhand der Figuren 1 und 2 dargestellten Vorrichtung unterscheidet.

Die in Figur 3 gezeigte Vorrichtung 10 unterscheidet sich von der anhand der Figuren 1 und 2 dargestellten Vorrichtung insbesondere dadurch, dass das zweite Objektiv 60 nicht starr mit dem ersten Objektiv 50 verbunden ist. Stattdessen ist zusätzlich zu dem großen und durch die erste Antriebseinrichtung 92 bewegbaren Schlitten 91 ein kleiner Schlitten 96 vorgesehen. Der kleine Schlitten 96 ist starr mit dem zweiten Objektiv 60 verbunden. Eine zweite Antriebseinrichtung 97 ist vorgesehen und ausgebildet, um den zweiten Schlitten 96 relativ zu dem ersten Schlitten 91 zu bewegen.

Die zweite Antriebseinrichtung 97 kann ausschließlich während des anhand der Figur 2 dargestellten Justiervorgangs verwendet werden. Alternativ oder zusätzlich können während der vorgesehenen Verwendung der Vorrichtung 10 der kleine Schlitten 96 und das zweite Objektiv 60 mittels der zweiten Antriebseinrichtung 97 relativ zu dem großen Schlitten 91 und dem ersten Objektiv 50 bewegt werden. Durch die Bewegung des kleinen Schlittens 96 und des zweiten Objektivs 60 relativ zu dem großen Schlitten und dem ersten Objektiv 50 können die zweite Fläche 80 und der zweite Schärfentiefenbereich 83 relativ zu der ersten Fläche 70 und zu dem ersten Schärfentiefenbereich 73 bewegt werden. Damit wird gleichzeitig der Überlapp zwischen den Schärfentiefenbereichen 73, 83 verändert. Der Überlapp zwischen den Schärfentiefenbereichen 73, 83 kann zur Anpassung an die Sehgewohnheiten oder Vorlieben eines Anwenders, an eine bestimmte Anwendungssituation und/oder an die Entfernung eines betrachteten Objekts von der Vorrichtung 10 verändert werden.

Bei den anhand der Figuren 1 bis 3 dargestellten Vorrichtungen 10 sind die Ebenen 33, 43, in denen die lichtempfindlichen Schichten 32, 42 der Bildsensoren 30, 40 liegen, identisch. Alternativ und abweichend von den Darstellungen in den Figuren 1 bis 3 können die Ebenen 33, 43, in denen die lichtempfindlichen Schichten 32, 42 der Bildsensoren 30, 40 liegen, parallel und voneinander beabstandet sein.

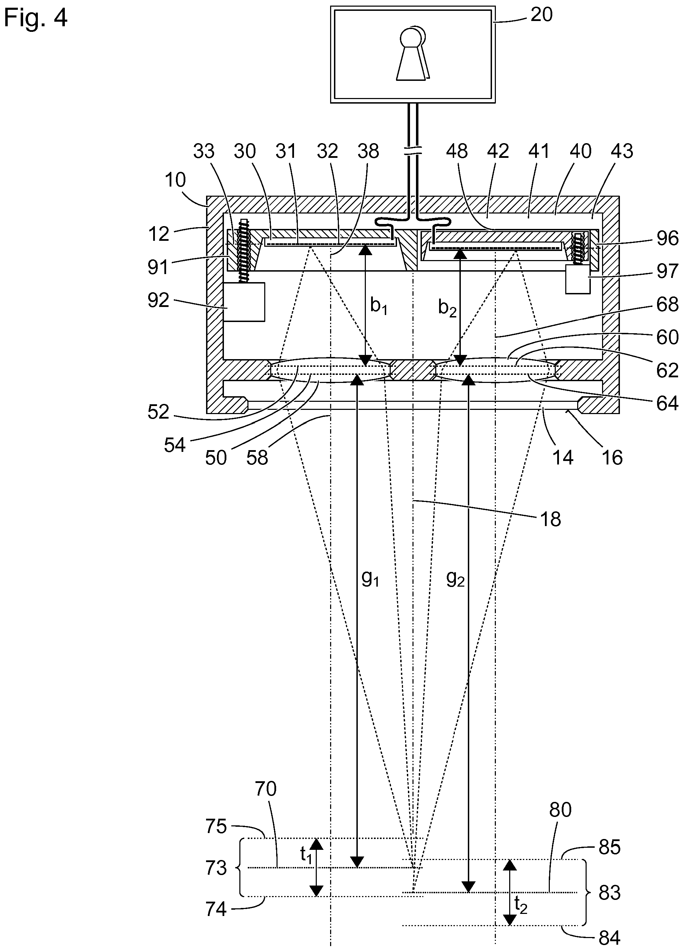

Figur 4 zeigt eine schematische Darstellung einer weiteren Vorrichtung 10 zum Erfassen eines Stereobilds, die in einigen Merkmalen, Eigenschaften und Funktionen den anhand der Figuren 1 bis 3 dargestellten Vorrichtungen ähnelt. Die Art der Darstellung in Figur 4 ähnelt der Art der Darstellung in den Figuren 1 und 3. Nachfolgend sind insbesondere Merkmale, Eigenschaften und Funktionen der Vorrichtung 10 beschrieben, in denen diese sich von den anhand der Figuren 1 bis 3 dargestellten Vorrichtungen unterscheidet.

Die in Figur 4 gezeigte Vorrichtung 10 unterscheidet sich von den anhand der Figuren 1 bis 3 dargestellten Vorrichtungen insbesondere dadurch, dass die Objektive 50, 60 starr mit dem Gehäuse 12 verbunden und die Bildsensoren 30, 40 relativ zu dem Gehäuse 12 bewegbar sind. Der erste Bildsensor 30 ist an einem großen Schlitten 91 starr befestigt, der mittels einer ersten Antriebseinrichtung 92 relativ zu dem Gehäuse 12 der Vorrichtung 10 und damit insbesondere relativ zu dem ersten Objektiv 50 bewegbar ist. Der zweite Bildsensor 40 ist an einem kleinen Schlitten 96 starr befestigt, der mittels einer zweiten Antriebseinrichtung 97 relativ zu dem gro-ßen Schlitten 91 bewegbar ist.

Durch die erste Antriebseinrichtung 92 können der Schlitten 91 und die Bildsensoren 30, 40 simultan bewegt werden, wodurch die Flächen 70, 80, die durch die Objektive 50, 60 scharf auf die Bildsensoren 30, 40 abgebildet werden, gleichzeitig bewegt werden. Der kleine Schlitten 96 und mit ihm der zweite Bildsensor 40 können durch die zweite Antriebseinrichtung 97 relativ zu dem großen Schlitten 91 bewegt werden, um die zweite Fläche 80, die durch das zweite Objektiv 60 scharf auf den zweiten Bildsensor 40 abgebildet wird, relativ zu der ersten Fläche 70, die durch das erste Objektiv 50 scharf auf den ersten Bildsensor 30 abgebildet wird, zu bewegen.

Bei dem in Figur 4 gezeigten Beispiel sind die objektseitigen Hauptebenen 54, 64 der Objektive 50, 60 identisch und die bildseitigen Hauptebenen 52, 62 der Objektive 50, 60 identisch. Alternativ und abweichend von der Darstellung in Figur 4 können die objektseitigen Hauptebenen 54, 64 der Objektive 50, 60 voneinander beabstandet und die bildseitigen Hauptebenen 52, 62 der Objektive 50, 60 voneinander beabstandet sein.

Alternativ und abweichend von der Darstellung in Figur 4 können bei Entfall des kleinen Schlittens 96 und der zweiten Antriebseinrichtung 97 beide Bildsensoren 30, 40 starr mit dem großen Schlitten 91 verbunden sein. Dabei können die Ebenen 33, 43, in denen die lichtempfindlichen Schichten 32, 42 der Bildsensoren 30, 40 liegen, parallel und voneinander beabstandet oder, wenn die Hauptebenen 52, 62, 54, 64 der Objektive 50, 60 abweichend von der Darstellung in Figur 4 nicht zusammenfallen, identisch sein.

Bei den anhand der Figuren 3 und 4 dargestellten Vorrichtungen 10 existiert eine klare funktionelle Trennung zwischen den Antriebseinrichtungen 92, 97. Die erste Antriebseinrichtung 92 ist jeweils zur gemeinsamen Bewegung der Flächen 70, 80 und der Schärfentiefenbereiche 73, 83 vorgesehen, die zweite Antriebseinrichtung 97 ist jeweils zur Bewegung der zweiten Fläche 80 relativ zu der ersten Fläche 70 und damit auch des zweiten Schärfentiefenbereichs 83 relativ zu dem ersten Schärfentiefenbereich 73 vorgesehen.

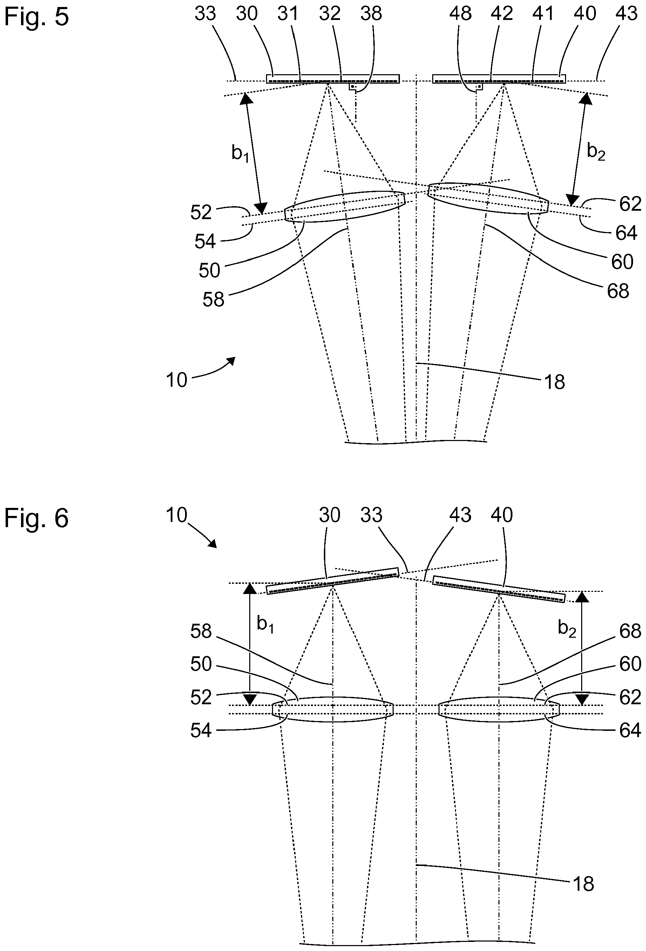

Figur 5 zeigt eine schematische Darstellung von Teilen einer weiteren Vorrichtung 10 zum Erfassen eines Stereobilds, die in einigen Merkmalen, Eigenschaften und Funktionen den anhand der Figuren 1 bis 4 dargestellten Vorrichtungen ähnelt. Die Art der Darstellung entspricht derjenigen der Darstellung in Figur 2. Insbesondere sind lediglich an der optischen Abbildung von Objekten unmittelbar beteiligte Bauteile der Vorrichtung 10 dargestellt, nämlich die Objektive 50, 60 und die Bildsensoren 30, 40. Nachfolgend sind insbesondere Merkmale, Eigenschaften und Funktionen der in Figur 5 gezeigten Vorrichtung 10 beschrieben, in denen diese sich von den anhand der Figuren 1 bis 4 dargestellten Vorrichtungen unterscheidet.

Die in Figur 5 gezeigte Vorrichtung 10 unterscheidet sich von den anhand der Figuren 1 bis 4 dargestellten Vorrichtungen insbesondere dadurch, dass die optischen Achsen 58, 68 der Objektive 50, 60 nicht parallel zueinander und zu der Hauptblickachse 18 der Vorrichtung 10 angeordnet sind. Vielmehr schneiden die optischen Achsen 58, 68 der Objektive 50, 60 die Hauptblickachse 18 der Vorrichtung 10 in einem Punkt außerhalb des dargestellten Raumbereichs. Entsprechend sind auch die bildseitigen Hauptebenen 52, 62 der Objektive 50, 60 nicht parallel zueinander, sondern schneiden einander in einer geraden Schnittlinie. Diese Schnittlinie ist bei dem dargestellten Beispiel orthogonal zu der Zeichenebene der Figur 5. Entsprechend sind auch die objektseitigen Hauptebenen 54, 64 der Objektive 50, 60 nicht parallel zueinander, sondern schneiden einander in einer geraden Schnittlinie. Auch diese Schnittlinie ist bei dem dargestellten Beispiel orthogonal zu der Zeichenebene der Figur 5.

Die Ebenen 33, 43, in denen die lichtempfindlichen Schichten 32, 42 der Bildsensoren 30, 40 angeordnet sind, sind nicht parallel zu den Hauptebenen 52, 54, 62, 64 der Objektive 50, 60 und nicht orthogonal zu den optischen Achsen 58, 68 der Objektive 50, 60. Der Abstand zwischen dem ersten Bildsensor 30 und der bildseitigen Hauptebene 52 des ersten Objektivs 50, d. h. die Bildweite b₁ für ein auf der optischen Achse 58 des ersten Objektivs 50 liegendes Objekt ist der Abstand zwischen dem Schnittpunkt der Ebene 33, in der die lichtempfindliche Schicht 32 des ersten Bildsensors 30 liegt, mit der optischen Achse 58 des ersten Objektivs 50 und dem Schnittpunkt der bildseitigen Hauptebene 52 des ersten Objektivs 50 mit der optischen Achse 58 des ersten Objektivs 50. Der Abstand zwischen dem zweiten Bildsensor 40 und der bildseitigen Hauptebene 62 des zweiten Objektivs 60, d. h. die Bildweite b₂ für ein auf der optischen Achse 68 des zweiten Objektivs 60 liegendes Objekt ist der Abstand zwischen dem Schnittpunkt der Ebene 43, in der die lichtempfindliche Schicht 42 des zweiten Bildsensors 40 liegt, mit der optischen Achse 68 des zweiten Objektivs 60 und dem Schnittpunkt der bildseitigen Hauptebene 62 des zweiten Objektivs 60 mit der optischen Achse 68 des zweiten Objektivs 60. Da die Ebenen 33, 43, in denen die lichtempfindlichen Schichten 32, 42 der Bildsensoren 30, 40 liegen, identisch und die Bildweiten b₁, b₂ unterschiedlich sind, liegt die Schnittlinie zwischen den bildseitigen Hauptebenen 52, 62 der Objektive 50, 60 asymmetrisch, schneidet also die Hauptblickachse 18 der Vorrichtung 10 nicht, sondern ist von dieser beabstandet.

Alternativ und abweichend von der Darstellung in Figur 5 können die Ebenen 33, 43, in denen die lichtempfindlichen Schichten 32, 42 der Bildsensoren 30, 40 liegen, parallel und voneinander beabstandet sein. In diesem Fall kann die Schnittlinie zwischen den bildseitigen Hauptebenen 52, 62 der Objektive 50, 60 die Hauptblickachse 18 der Vorrichtung 10 schneiden.

Figur 6 zeigt eine schematische Darstellung einer weiteren Vorrichtung 10 zum Erfassen eines Stereobilds, die in einigen Merkmalen, Eigenschaften und Funktionen den anhand der Figuren 1 bis 5 dargestellten Vorrichtungen ähnelt. Die Art der Darstellung in Figur 6 entspricht derjenigen der Darstellung in Figur 5. Nachfolgend sind insbesondere Merkmale, Eigenschaften und Funktionen beschrieben, in denen die in Figur 6 gezeigte Vorrichtung 10 sich von den anhand der Figu-ren 1 bis 5 dargestellten Vorrichtungen unterscheidet.

Bei der in Figur 6 gezeigten Vorrichtung 10 sind die bildseitigen Hauptebenen 52, 62 der Objektive 50, 60 identisch, und die objektseitigen Hauptebenen 54, 64 der Objektive 50, 60 sind identisch. Die optischen Achsen 58, 68 der Objektive 50, 60 sind parallel zueinander und parallel zu der Hauptblickachse 18 der Vorrichtung 10. Die Ebenen 33, 43, in denen die lichtempfindlichen Schichten 32, 42 der Bildsensoren 30, 40 liegen, sind nicht orthogonal zu den optischen Achsen 58, 68 der Objektive 50, 60 und nicht parallel zueinander. Die Bildweiten b₁, b₂ sind unterschiedlich. Bei dem dargestellten Beispiel schließen die Flächennormalen der Ebenen 33, 43, in denen die lichtempfindlichen Schichten 32, 42 der Bildsensoren 30, 40 liegen, mit den optischen Achsen 58, 68 der Objektive 50, 60 gleiche Winkel ein. Die Ebenen 33, 43, in denen die lichtempfindlichen Schichten 32, 42 der Bildsensoren 30, 40 liegen, schneiden sich in einer geraden Schnittlinie. Diese Schnittlinie ist bei dem dargestellten Beispiel orthogonal zu der Zeichenebene der Figur 6. Diese Schnittlinie schneidet die Hauptblickachse 18 der Vorrichtung 10 nicht, sondem ist von dieser beabstandet.

Alternativ und abweichend von der Darstellung in Figur 6 können die bildseitigen Hauptebenen 52, 62 der Objektive 50, 60 von einander verschieden, insbesondere parallel zu einander und von einander beabstandet sein. In diesem Fall kann die Schnittlinie zwischen den Ebenen 33, 43, in denen die lichtempfindlichen Schichten 32, 42 der Bildsensoren 30, 40 liegen, die Hauptblickachse 18 der Vorrichtung 10 schneiden.

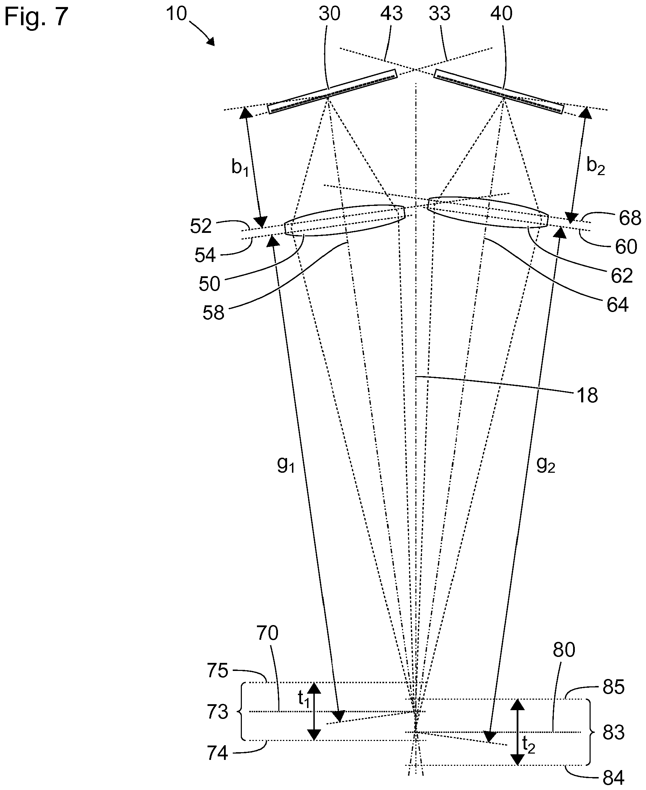

Figur 7 zeigt eine schematische Darstellung einer weiteren Vorrichtung 10, die in einigen Merkmalen, Eigenschaften und Funktionen den anhand der Figuren 1 bis 6 dargestellten Vorrichtungen ähnelt. Die Art der Darstellung entspricht derjenigen der Darstellungen in den Figuren 5 und 6. Nachfolgend sind insbesondere Merkmale, Eigenschaften und Funktionen beschrieben, in denen die in Figur 7 gezeigte Vorrichtung 10 sich von den anhand der Figuren 1 bis 6 dargestellten Vorrichtungen unterscheidet.

Die in Figur 7 gezeigte Vorrichtung 10 unterscheidet sich von den anhand der Figuren 1 bis 6 dargestellten Vorrichtungen insbesondere dadurch, dass - ähnlich wie bei der anhand der Figur 5 dargestellten Vorrichtung - die optischen Achsen 58, 68 der Objektive 50, 60 nicht parallel zueinander sind, sondern einander schneiden, und dass gleichzeitig die Ebenen 33, 43, in denen die lichtempfindlichen Schichten 32, 42 der Bildsensoren 30, 40 liegen, nicht parallel zueinander sind, sondern sich - ähnlich wie bei der anhand der Figur 6 dargestellten Vorrichtung - in einer geraden Schnittlinie schneiden. Diese Schnittlinie ist bei dem dargestellten Beispiel orthogonal zu der Zeichenebene der Figur 7. Entsprechend schneiden sich auch die bildseitigen Hauptebenen 52, 62 der Objektive 50, 60 in einer geraden Schnittlinie. Auch diese Schnittlinie ist bei dem dargestellten Beispiel orthogonal zu der Zeichenebene der Figur 7. Die Bildweiten b₁, b₂ unterscheiden sich.

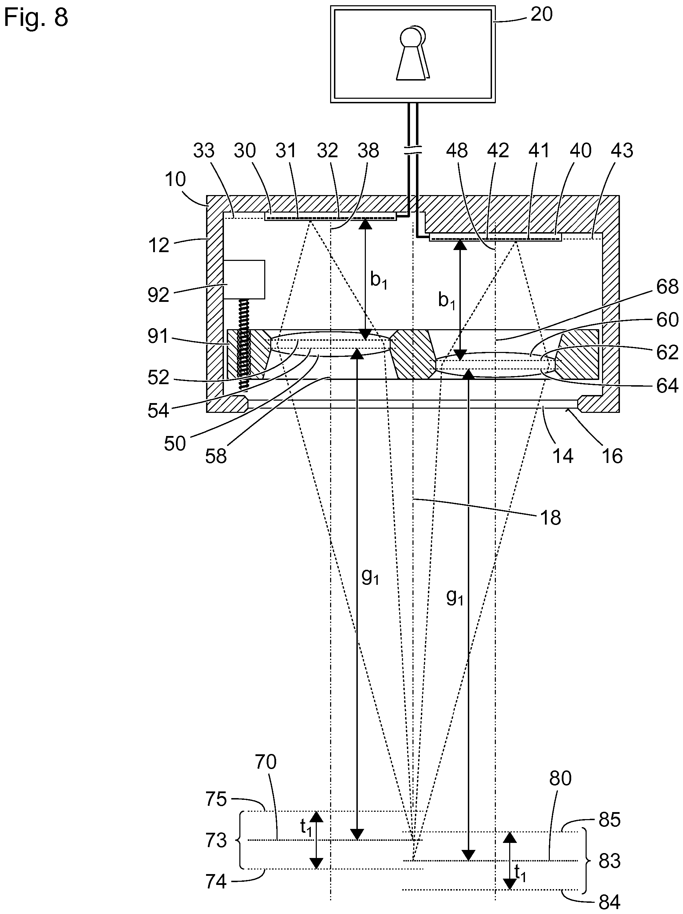

Figur 8 zeigt eine schematische Darstellung einer weiteren erfindungsgemäßen Vorrichtung 10 zum Erfassen eines Stereobilds, die in einigen Merkmalen, Eigenschaften und Funktionen den anhand der Figuren 1 bis 7 dargestellten Vorrichtungen und besonders der anhand der Figuren 1 und 2 dargestellten Vorrichtung ähnelt. Die Art der Darstellung in Figur 8 ähnelt der Art der Darstellung in den Figuren 1, 3 und 4. Nachfolgend sind insbesondere Merkmale, Eigenschaften und Funktionen der Vorrichtung 10 beschrieben, in denen diese sich von der anhand der Figuren 1 und 2 dargestellten Vorrichtung unterscheidet.

Die in Figur 8 gezeigte Vorrichtung 10 unterscheidet sich von der anhand der Figuren 1 und 2 dargestellten Vorrichtung insbesondere dadurch, dass nicht nur beide Objektive 50, 60, sondern auch beide Bildsensoren 30, 40 relativ zu einander in einer Richtung parallel zu der Hauptblickachse 18 versetzt angeordnet sind. Der zweite Bildsensor 40 ist relativ zu dem ersten Bildsensor 30 um die gleiche Distanz versetzt angeordnet, um die auch das zweite Objektiv 60 relativ zu dem ersten Objektiv 50 versetzt angeordnet ist. Die Anordnung aus dem ersten Bildsensor 30 und dem ersten Objektiv 50 und die Anordnung aus dem zweiten Bildsensor 40 und dem zweiten Objektiv 60 weisen deshalb die gleiche Bildweite b₁ auf.

Beide Objektive 50, 60 sind - im Rahmen der Fertigungstoleranz bzw. der Serienstreuung - gleich oder weisen zumindest die gleiche Brennweite auf. Deshalb weisen die Anordnung aus dem ersten Bildsensor 30 und dem ersten Objektiv 50 und die Anordnung aus dem zweiten Bildsensor 40 und dem zweiten Objektiv 60 auch die gleiche Gegenstandsweite g₁ auf. Entsprechend sind die Flächen 70, 80, die durch die Objektive 50, 60 scharf in die lichtempfindlichen Schichten 32, 42 der Bildsensoren 30, 40 abgebildet werden, um die gleiche Distanz versetzt, um die die Bildsensoren 30, 40 und die Objektive 50, 60 relativ zu einander versetzt sind.

Die Distanz, um die der zweite Bildsensor 40 relativ zu dem ersten Bildsensor 30 und das zweite Objektiv 60 relativ zu dem ersten Objektiv 50 und die zweite Fläche 80 relativ zu der ersten Fläche 70 versetzt sind, ist jedoch so gewählt, dass die Schärfentiefenbereiche 73, 83 überlappen.

Alternativ und abweichend von der Darstellung in Figur 8 kann bei der Vorrichtung 10 - beispielsweise ähnlich wie bei den anhand der Figuren 3 und 4 dargestellten Ausführungsformen - das zweite Objektiv 60 relativ zu dem ersten Objektiv 50 und/oder der zweite Bildsensor 40 relativ zu dem ersten Bildsensor 30 bewegbar sein.

Alternativ und abweichend von der Darstellung in Figur 8 können bei der Vorrichtung 10 - beispielsweise ähnlich wie bei den anhand der Figuren 5 bis 7 dargestellten Ausführungsformen - die Ebenen 33, 43, in denen die lichtempfindlichen Schichten 32, 42 der Bildsensoren 30, 40 liegen und/oder die Hauptebenen 52, 54, 62, 64 der Objektive 50, 60 nicht orthogonal zu der Hauptblickachse 18, sondern geneigt angeordnet sein. Dabei sind jedoch die Anordnung aus dem ersten Bildsensor 30 und dem ersten Objektiv 50 und die Anordnung aus dem zweiten Bildsensor 40 und dem zweiten Objektiv 60 - von dem Versatz in Richtung parallel zu der Hauptblickachse 18 abgesehen - insbesondere spiegelsymmetrisch zu einander.

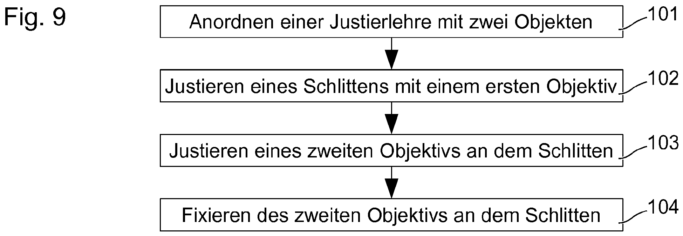

Figur 9 zeigt ein schematisches Flussdiagramm eines Verfahrens zum Justieren einer Vorrichtung zum Erfassen eines Stereobilds. Das Verfahren ist insbesondere anwendbar auf die anhand der Figuren 1 bis 8 dargestellten Vorrichtungen 10. Das Verfahren ist jedoch auch anwendbar auf Vorrichtungen, die sich von den anhand der Figuren 1 bis 8 dargestellten Vorrichtungen unterscheiden. Dennoch werden nachfolgend Bezugszeichen aus den Figuren 1 bis 8 verwendet, um ein Verständnis des Verfahrens zu vereinfachen.

Bei einem ersten Schritt 101 wird eine Justierlehre 93 mit einer ersten Fläche 94 und einer zweiten Fläche 95 in einer vorbestimmten Position und Orientierung relativ zu der zu justierenden Vorrichtung 10 angeordnet. Die erste Fläche 94 und die zweite Fläche 95 an der Justierlehre 93 liegen jeweils insbesondere in einer Ebene, wobei beide Ebenen zueinander parallel und voneinander beabstandet sind. Die Flächen 94, 95 an der Justierlehre 93 sind optisch kontraststark strukturiert, um eine Fokussierung auf die Flächen 94, 95 zu vereinfachen.

Bei einem zweiten Schritt 102 wird ein erstes Objektiv 50 der Vorrichtung 10 so justiert, dass das erste Objektiv 50 ein - bis auf Beugung an der Aperturblende des ersten Objektivs 50 und Abbildungsfehler - scharfes Bild der ersten Fläche 94 an der Justierlehre 93 in der lichtempfindlichen Schicht 32 eines ersten Bildsensors 30 erzeugt. Der zweite Schritt 102 erfolgt insbesondere durch Bewegen eines Schlittens 91, mit dem das erste Objektiv 50 starr verbunden ist.

Bei einem dritten Schritt 103 wird ein zweites Objektiv 60 so justiert, dass das zweite Objektiv 60 ein - bis auf Beugung an der Aperturblende des zweiten Objektivs 60 und Abbildungsfehler - scharfes Bild der zweiten Fläche 95 an der Justierlehre 93 in der lichtempfindlichen Schicht 42 eines zweiten Bildsensors 40 erzeugt. Der dritte Schritt 103 wird insbesondere ausgeführt durch Bewegen des zweiten Objektivs 60 relativ zu dem Schlitten 91.

Bei einem optionalen vierten Schritt 104 wird das zweite Objektiv 60 relativ zu dem ersten Objektiv 50 fixiert, insbesondere durch Fixieren bzw. starres mechanisches Verbinden des zweiten Objektivs 60 mit dem Schlitten 91.

**Bezugszeichen**

| | |
|---|---|
| **10** | **Vorrichtung** zum Erfassen eines Stereobilds |
| 12 | Gehäuse der Vorrichtung 10 |
| 14 | Fensterbauteil der Vorrichtung 10 |
| 16 | Lichteintrittsfläche der Vorrichtung 10 an dem Fensterbauteil 14 |
| 18 | Hauptblickachse der Vorrichtung 10 |
| 20 | Wiedergabevorrichtung zum Wiedergeben eines von der Vorrichtung 10 erfassten Stereobilds |
| **30** | **erster Bildsensor** der Vorrichtung 10 |
| 31 | lichtempfindliche Zelle des ersten Bildsensors 30 |
| 32 | lichtempfindliche Schicht des ersten Bildsensors 30 |
| 33 | Ebene, in der die lichtempfindliche Schicht 32 des ersten Bildsensors 30 liegt |
| 38 | Flächennormale der Ebene 33, in der die lichtempfindliche Schicht 32 liegt |
| **40** | **zweiter Bildsensor** der Vorrichtung 10 |
| 41 | lichtempfindliche Zelle des zweiten Bildsensors 40 |
| 42 | lichtempfindliche Schicht des zweiten Bildsensors 40 |
| 43 | Ebene, in der die lichtempfindliche Schicht 42 des zweiten Bildsensors 40 liegt |
| 48 | Flächennormale der Ebene 43, in der die lichtempfindliche Schicht 42 liegt |
| **50** | **erstes Objektiv** der Vorrichtung 10 |
| 52 | bildsseitige Hauptebene des ersten Objektivs 50 |
| 54 | objektseitige Hauptebene des ersten Objektivs 50 |
| 58 | optische Achse des ersten Objektivs 50 der Vorrichtung 10 |
| **60** | **zweites Objektiv** der Vorrichtung 10 |
| 62 | bildsseitige Hauptebene des zweiten Objektivs 60 |
| 64 | objektseitige Hauptebene des zweiten Objektivs 60 |
| 68 | optische Achse des zweiten Objektivs 60 der Vorrichtung 10 |
| **70** | **erste Fläche**, die durch das erste Objektiv 50 scharf auf den ersten Bildsensor 30 abgebil-det wird |
| 73 | erster Schärfentiefenbereich der Anordnung aus dem ersten Bildsensor 30 und dem erstenObjektiv 50 |
| 74 | distaler Rand des ersten Schärfentiefenbereichs 73 |
| 75 | proximaler Rand des ersten Schärfentiefenbereichs 73 |
| **80** | **zweite Fläche**, die durch das zweite Objektiv 60 scharf auf den zweiten Bildsensor 40 ab-gebildet wird |
| 83 | zweiter Schärfentiefenbereich der Anordnung aus dem zweiten Bildsensor 40 und demzweiten Objektiv 60 |
| 84 | distaler Rand des zweiten Schärfentiefenbereichs 83 |
| 85 | proximaler Rand des zweiten Schärfentiefenbereichs 83 |
| 91 | (großer) Schlitten |
| 92 | (erste) Antriebseinrichtung zum Bewegen des (großen) Schlittens 91 |
| 93 | Justierlehre |
| 94 | erste Fläche an der Justierlehre 93 |
| 95 | zweite Fläche an der Justierlehre 93 |
| 96 | kleiner Schlitten |
| 97 | zweite Antriebseinrichtung zum Bewegen des kleinen Schlittens 96 relativ zu dem großen Schlitten 91 |
| 101 | erster Schritt (Anordnen einer Justierlehre mit einem ersten Objekt und einem zweiten Ob-jekt an einer vorbestimmten Position) |
| 102 | zweiter Schritt (Justieren eines Schlittens derart, dass ein erstes Objektiv an dem Schlittenein scharfes Bild des ersten Objekts auf einem ersten Bildsensor erzeugt) |
| 103 | dritter Schritt (Justieren eines zweiten Objektivs relativ zu dem Schlitten derart, dass daszweite Objektiv ein scharfes Bild des zweiten Objekts auf einem zweiten Bildsensor er-zeugt) |
| 104 | vierter Schritt (Fixieren des zweiten Objektivs an dem Schlitten |
| b₁ | Bildweite der Anordnung aus dem ersten Bildsensor 30 und dem ersten Objektiv 50 = Ab-stand der lichtempfindlichen Schicht 32 des ersten Bildsensors 30 von der bildseitigenHauptebene 52 des ersten Objektivs 50 |
| g₁ | Objektweite der Anordnung aus dem ersten Bildsensor 30 und dem ersten Objektiv 50 =Abstand der ersten Fläche 70 von der objektseitigen Hauptebene 52 des ersten Objektivs50 |
| t₁ | Tiefe des ersten Schärfentiefenbereichs 73 der Anordnung aus dem erstem Bildsensor 30und dem ersten Objektiv 50 |
| b₂ | Bildweite der Anordnung aus dem zweiten Bildsensor 40 und dem zweiten Objektiv 60 = Abstand der lichtempfindlichen Schicht 42 des zweiten Bildsensors 40 von der bildseitigen Hauptebene 62 des zweiten Objektivs 60 |
| g₂ | Objektweite der Anordnung aus dem zweiten Bildsensor 40 und dem zweiten Objektiv 60 = Abstand der zweiten Fläche 80 von der objektseitigen Hauptebene 62 des zweiten Objektivs 60 |
| t₂ | Tiefe des zweiten Schärfentiefenbereichs 83 der Anordnung aus dem zweiten Bildsensor 40 und dem zweiten Objektiv 60 |

## Patentansprüche

1. **Vorrichtung** (10) zum Erfassen eines Stereobilds, mit:
einem **ersten Bildsensor** (30) mit einer ersten lichtempfindlichen Schicht (32) zum Erfassen eines ersten Bilds;
einem **zweiten Bildsensor** (40) mit einer zweiten lichtempfindlichen Schicht (42) zum Erfassen eines zweiten Bilds;
einem **ersten Objektiv** (50) mit einer ersten bildseitigen Hauptebene (52), das Punkte, die in einer **ersten Fläche** (70) liegen, scharf auf die erste lichtempfindliche Schicht (32) des ersten Bildsensors (30) abbildet;
einem **zweiten Objektiv** (60) mit einer zweiten bildseitigen Hauptebene (62), das Punkte, die in einer **zweiten Fläche** (80) liegen, scharf auf die zweite lichtempfindliche Schicht (42) des zweiten Bildsensors (40) abbildet,
wobei der **Abstand der** ersten **bildseitigen Hauptebene** (52) des ersten Objektivs (50) **von der** ersten **lichtempfindlichen Schicht** (32) des ersten Bildsensors (30) und der Abstand der zweiten bildseitigen Hauptebene (62) des zweiten Objektivs (60) von der zweiten lichtempfindlichen Schicht (42) des zweiten Bildsensors (40) **verschieden** sind, so dass die erste Fläche (70) und die zweite Fläche (80) beabstandet oder in Richtung parallel zur Hauptblickachse (18) der Vorrichtung (10) versetzt sind,
ferner mit:
einem relativ zu den Bildsensoren (30, 40) bewegbaren **Schlitten** (91), an dem das erste **Objektiv** (50) starr oder in einer Richtung, die nicht parallel zu der optischen Achse (58) des Objektivs (50) ist, bewegbar befestigt ist, und an dem das zweite **Objektiv** (60) starr oder bewegbar befestigt ist;
einer **Schlitten-Antriebseinrichtung** (92) zum Bewegen des Schlittens (91) zum gleichzeitigen Bewegen der ersten Fläche (70) und der zweiten Fläche (80).

2. Vorrichtung (10) nach dem vorangehenden Anspruch, ferner mit:
einer **Objektiv-Antriebseinrichtung** (97) zum Bewegen des zweiten Objektivs (60) oder eines Teils des zweiten Objektivs (60) relativ zu dem Schlitten (91) und zu dem ersten Objektiv (50) und zum Bewegen der zweiten Fläche (80) relativ zu der ersten Fläche (70).

3. Vorrichtung (10) nach einem der vorangehenden Ansprüche, bei der
die lichtempfindlichen Schichten (32, 42) der **Bildsensoren in derselben Ebene** (33, 43) liegen;
die erste bildseitige Hauptebene (52) des ersten Objektivs (50) und die zweite bildseitige Hauptebene (62) des zweiten Objektivs (60) parallel und von einander beabstandet sind oder die Schnittlinie der bildseitigen Hauptebenen (52, 62) der Objektive (50, 60) nicht die Hauptblickachse (18) der Vorrichtung (10) schneidet.

4. Vorrichtung (10) nach einem der Ansprüche 1 und 2, bei der
die erste bildseitige Hauptebene (52) des ersten Objektivs (50) und die zweite bildseitige Hauptebene (62) des zweiten Objektivs (60) identisch sind,
die Bildsensoren (30, 40) so angeordnet sind, dass die **lichtempfindlichen Schichten** (32, 42) der Bildsensoren (30, 40) in zwei **parallelen und von einander beabstandeten Ebenen** (33, 43) liegen.

5. Vorrichtung (10) nach einem der vorangehenden Ansprüche, bei der
das erste Objektiv (50) und das zweite Objektiv (60) die **gleiche Brennweite** aufweisen.

6. **Vorrichtung** (10) zum Erfassen eines Stereobilds, mit:
einem **ersten Bildsensor** (30) mit einer ersten lichtempfindlichen Schicht (32) zum Erfassen eines ersten Bilds;
einem **zweiten Bildsensor** (40) mit einer zweiten lichtempfindlichen Schicht (42) zum Erfassen eines zweiten Bilds;
einem **ersten Objektiv** (50) mit einer ersten bildseitigen Hauptebene (52), das Punkte, die in einer **ersten Fläche** (70) liegen, scharf auf die erste lichtempfindliche Schicht (32) des ersten Bildsensors (30) abbildet;
einem **zweiten Objektiv** (60) mit einer zweiten bildseitigen Hauptebene (62), das Punkte, die in einer **zweiten Fläche** (80) liegen, scharf auf die zweite lichtempfindliche Schicht (42) des zweiten Bildsensors (40) abbildet,
wobei das **erste Objektiv** (50) und das **zweite Objektiv** (60) einander **gleichen,**
wobei der Abstand des ersten Objektivs (50) von dem ersten Bildsensor (30) dem Abstand des zweiten Objektivs (60) von dem zweiten Bildsensor (40) gleicht,
wobei das erste Objektiv (50) und das zweite Objektiv (60) in Richtung der Hauptblickachse (18) der Vorrichtung (10) **versetzt angeordnet** sind, so dass die erste Fläche (70) und die zweite Fläche (80) beabstandet oder in Richtung parallel zur Hauptblickachse (18) der Vorrichtung (10) versetzt sind,
ferner mit:
einem relativ zu den Bildsensoren (30, 40) bewegbaren **Schlitten** (91), an dem das erste **Objektiv** (50) starr oder in einer Richtung, die orthogonal zu der optischen Achse (58) des Objektivs (50) ist, bewegbar befestigt ist, und an dem das zweite **Objektiv** (60) starr befestigt ist;
einer **Schlitten-Antriebseinrichtung** (92) zum Bewegen des Schlittens (91) zum gleichzeitigen Bewegen der ersten Fläche (70) und der zweiten Fläche (80).

7. Vorrichtung (10) nach einem der vorangehenden Ansprüche, bei der
das erste Objektiv (50) und der erste Bildsensor (30) einen **ersten Schärfentiefenbereich** (73) aufweisen,
das zweite Objektiv (60) und der zweite Bildsensor (40) einen **zweiten Schärfentiefenbereich** (83) aufweisen,
der erste **Schärfentiefenbereich** (73) und der zweite Schärfentiefenbereich (83) **überlappen.**

8. Vorrichtung (10) nach dem vorangehenden Anspruch, bei der
die **erste Fläche** (70) **innerhalb des zweiten Schärfentiefenbereichs** (83) liegt,
die zweite Fläche (80) innerhalb des ersten Schärfentiefenbereichs (73) liegt.

9. Vorrichtung (10) nach einem der Ansprüche 7 oder 8, bei der
die Schärfentiefenbereiche (73, 83) zu mindestens **der Hälfte** der Tiefe t₁ des ersten Schärfentiefenbereichs (73) und zu mindestens der Hälfte der Tiefe t₂ des zweiten Schärfentiefenbereichs (83) und zu höchstens **zwei Dritteln** der Tiefe t₁ des ersten Schärfentiefenbereichs (73) und zu höchstens zwei Dritteln der Tiefe t₂ des zweiten Schärfentiefenbereichs (83) **überlappen.**

10. **Verfahren zum Justieren** einer Vorrichtung (10) nach einem der vorangehenden Ansprüche bei Herstellung oder Wartung der Vorrichtung (10), mit folgenden Schritten:
**Anordnen** (101) einer **Justierlehre** (93) mit einem ersten Objekt (50) und mit einem zweiten Objekt (60) an einer vorbestimmten Position relativ zu der Vorrichtung (10) derart, dass das erste Objekt (94) in einer ersten vorbestimmten Ebene (70) und das zweite Objekt (95) in einer zweiten vorbestimmten Ebenen (80) angeordnet sind, wobei die erste Ebene (70) und die zweite Ebene (80) jeweils **orthogonal zu einer Hauptblickachse** (18) der Vorrichtung (10) sind, wobei die erste vorbestimmte Ebene (70) und die zweite vorbestimmte Ebene (80) parallel zu einander und von einander beabstandet sind;
**Justieren** (102) zumindest entweder eines ersten Objektivs (50) oder eines ersten Bildsensors (30) der Vorrichtung (10) derart, dass das erste Objektiv (50) ein **scharfes Bild des ersten Objekts** (94) an dem ersten Bildsensor (30) erzeugt;
**Justieren** (103) zumindest entweder eines zweiten Objektivs (60) oder eines zweiten Bildsensors (40) der Vorrichtung (10) derart, dass das zweite Objektiv (60) ein **scharfes Bild des zweiten Objekts** (95) an dem zweiten Bildsensor (40) erzeugt.

## Claims

1. **Device** (10) for capturing a stereoscopic image, comprising:
a **first image sensor** (30) comprising a first light-sensitive layer (32) for capturing a first image;
a **second image sensor** (40) comprising a second light-sensitive layer (42) for capturing a second image;
a **first lens** (50) comprising a first image-side principal plane (52), which first lens (50) images points lying in a **first area** (70) sharply onto the first light-sensitive layer (32) of the first image sensor (30);
a **second lens (60)** comprising a second image-side principal plane (62), which second lens (60) images points lying in a **second area** (80) sharply onto the second light-sensitive layer (42) of the second image sensor (40),
wherein **the distance** between **the first image-side principal plane** (52) of the first lens (50) and the **first light-sensitive layer** (32) of the first image sensor (30) and the distance between the second image-side principal plane (62) of the second lens (60) and the second light-sensitive layer (42) of the second image sensor (40) are **different,** such that the first area (70) and the second area (80) are spaced apart or offset in a direction parallel to the principal viewing axis (18) of the device (10),
the device (10) further comprising:
a **slide** (91) movable relative to the image sensor (30, 40), at which slide (91) the first **lens** (50) is secured rigidly or movably in a direction that is not parallel to the optical axis (58) of the lens (50), and at which the second **lens** (60) is secured rigidly or movably;
a **slide drive** (92) for moving the slide (91) for simultaneously moving the first area (70) and the second area (80).

2. Device (10) according to the preceding claim, furthermore comprising:
a **lens drive** (97) for moving the second lens (60) or a part of the second lens (60) relative to the slide (91) and to the first lens (50) and for moving the second area (80) relative to the first area (70).

3. Device (10) according to one of the preceding claims, wherein
the light-sensitive layers (32, 42) of the **image sensors** lie **in the same plane** (33, 43);
the first image-side principal plane (52) of the first lens (50) and the second image-side principal plane (62) of the second lens (60) are parallel and spaced apart from one another or the line of intersection of the image-side principal planes (52, 62) of the lenses (50, 60) does not intersect the principal viewing axis (18) of the device (10).

4. Device (10) according to one of the claims 1 and 2, wherein
the first image-side principal plane (52) of the first lens (50) and the second image-side principal plane (62) of the second lens (60) are identical,
the image sensors (30, 40) are arranged such that the **light-sensitive layers** (32, 42) of the image sensors (30, 40) lie in two **planes** (33, 43) **which are parallel and spaced apart from one another.**

5. Device (10) according to one of the preceding claims, wherein
the first lens (50) and the second lens (60) have the **same focal length.**

6. **Device** (10) for capturing a stereoscopic image, comprising:
a **first image sensor** (30) with a first light-sensitive layer (32) for capturing a first image;
a **second image sensor** (40) with a second light-sensitive layer (42) for capturing a second image;
a **first lens** (50) with a first image-side principal plane (52), which first lens (50) images points lying in a **first area** (70) sharply onto the first light-sensitive layer (32) of the first image sensor (30);
a **second lens** (60) with a second image-side principal plane (62), which second lens (60) images points lying in a **second area** (80) sharply onto the second light-sensitive layer (42) of the second image sensor (40),
wherein the **first lens** (50) and the **second lens** (60) are **similar,**
the **distance** between the first lens (50) and the first image sensor (30) and the distance between the second lens (60) and the second image sensor (40) are similar,
the first lens (50) and the second lens (60) are **offset** in the direction of the principal viewing axis (18) of the device (10), such that the first area (70) and the second area (80) are spaced apart or offset in the direction of the principal viewing direction (18) of the device (10),
the device further comprising:
a **slide** (91) movable relative to the image sensors (30, 40), at which slide (91) the first **lens** (50) is secured rigidly or movably in a direction that is orthogonal to the optical axis (58) of the lens (50), and at which slide (91) the second **lens** (60) is secured rigidly;
a **slide drive** (92) for moving the slide (91) for simultaneously moving the first area (70) and the second area (80).

7. Device (10) according to one of the preceding claim, wherein
the first lens (50) and the first image sensor (30) provide a **first depth of field range** (73),
the second lens (60) and the second image sensor (40) provide a **second depth of field range** (83),
the first **depth of field range** (73) and the second depth of field range (83) **overlap.**

8. Device (10) according to the preceding claim, wherein
the **first area** (70) lies **within the second depth of field range** (83),
the second area (80) lies within the first depth of field range (73).

9. Device (10) according to one of the claims 7 and 8, wherein
the depth of field ranges (73, 83) **overlap** by at least half of the depth t₁ of the first depth of field range (73) and by at least **half** of the depth t₂ of the second depth of field range (83) and by at most two thirds of the depth t₁ of the first depth of field range (73) and by at most **two thirds** of the depth t₂ of the second depth of field range (83).

10. **Method for adjusting** a device (10) according to one of the preceding claims during production or maintenance of the device (10), comprising the following steps:
**arranging** (101) an **adjustment gauge** (93) with a first object (50) and a second object (60) at a predetermined position relative to the device (10) in such a way that the first object (94) is arranged in a first predetermined plane (70) and the second object (95) is arranged in a second predetermined plane (80), wherein both the first plane (70) and the second plane (80) are **orthogonal to a principal viewing axis** (18) of the device (10), wherein the first predetermined plane (70) and the second predetermined plane (80) are parallel to one another and spaced apart from one another;
**adjusting** (102) at least one of a first lens (50) and a first image sensor (30) of the device (10) in such a way that the first lens (50) generates a **sharp image of the first object** (94) at the first image sensor (30);
**adjusting** (103) at least one of a second lens (60) and a second image sensor (40) of the device (10) in such a way that the second lens (60) generates a **sharp image of the second object** (95) at the second image sensor (40).

## Revendications

1. **Dispositif** (10) de capture d'une image stéréo, ledit dispositif comprenant :
un **premier capteur d'images** (30) pourvu d'une première couche photosensible (32) destinée à capturer une première image ;
un **deuxième capteur d'images** (40) pourvu d'une deuxième couche photosensible (42) destinée à capturer une deuxième image ;
un **premier objectif** (50) comportant un premier plan principal côté image (52) qui reproduit des points, qui sont situés dans une **première surface** (70), de manière nette sur la première couche photosensible (32) du premier capteur d'images (30) ;
un **deuxième objectif** (60) comportant un deuxième plan principal côté image (62) qui reproduit des points, qui sont situés dans une deuxième surface (80), de manière nette sur la deuxième couche photosensible (42) du deuxième capteur d'images (40),
la **distance** du premier **plan principal côté image** (52) du premier objectif (50) **à la** première **couche photosensible** (32) du premier capteur d'images (30) et la distance du deuxième plan principal côté image (62) du deuxième objectif (60) à la deuxième couche photosensible (42) du deuxième capteur d'images (40) étant **différentes** de sorte que la première surface (70) et la deuxième surface (80) soient espacées ou décalées dans une direction parallèle à l'axe de vision principal (18) du dispositif (10),
et comprenant en outre :
un **chariot** (91) mobile par rapport aux capteurs d'image (30, 40), sur lequel le premier **objectif** (50) est monté de manière fixe ou mobile dans une direction qui n'est pas parallèle à l'axe optique (58) de l'objectif (50), et sur lequel le deuxième **objectif** (60) est monté de manière fixe ou mobile
un **module d'entraînement de chariot** (92) destiné à déplacer le chariot (91) afin de déplacer en même temps la première surface (70) et la deuxième surface (80).

2. Dispositif (10) selon la revendication précédente, comprenant en outre :
un **module d'entraînement d'objectif** (97) destiné à déplacer le deuxième objectif (60) ou une partie du deuxième objectif (60) par rapport au chariot (91) et au premier objectif (50) et déplacer la deuxième surface (80) par rapport à la première surface (70).

3. Dispositif (10) selon l'une des revendications précédentes, dans lequel
les couches photosensibles (32, 42) des **capteurs d'images sont situées dans le même plan** (33, 43) ;
le premier plan principal côté image (52) du premier objectif (50) et le deuxième plan principal côté image (62) du deuxième objectif (60) sont parallèles l'un à l'autre et espacés l'un de l'autre, ou la ligne d'intersection des plans principaux côté image (52, 62) des objectifs (50, 60) ne coupe pas l'axe de vision principal (18) du dispositif (10).

4. Dispositif (10) selon l'une des revendications 1 et 2, dans lequel
le premier plan principal côté image (52) du premier objectif (50) et le deuxième plan principal côté image (62) du deuxième objectif (60) sont identiques,
les capteurs d'images (30, 40) sont disposés de sorte que les **couches photosensibles** (32, 42) des capteurs d'images (30, 40) soient situées dans deux plans parallèles (33, 43) es**pacés l'un de l'autre.**

5. Dispositif (10) selon l'une des revendications précédentes, dans lequel le premier objectif (50) et le deuxième objectif (60) ont la **même distance focale.**

6. **Dispositif** (10) de capture d'une image stéréo, ledit dispositif comprenant :
un **premier capteur d'images** (30) pourvu d'une première couche photosensible (32) destinée à capturer une première image ;
un **deuxième capteur d'images** (40) pourvu d'une deuxième couche photosensible (42) destinée à capturer une deuxième image ;
un **premier objectif** (50) comportant un premier plan principal côté image (52) qui reproduit des points, qui sont situés dans une première surface (70), de manière nette sur la première couche photosensible (32) du premier capteur d'images (30) ;
un **deuxième objectif** (60) comportant un deuxième plan principal côté image (62) qui reproduit des points, qui sont situés dans une deuxième surface (80), de manière nette sur la deuxième couche photosensible (42) du deuxième capteur d'images (40),
le **premier objectif** (50) et le **deuxième objectif** (60) étant **identiques,**
la distance du premier objectif (50) au premier capteur d'images (30) étant égale à la distance du deuxième objectif (60) au deuxième capteur d'images (40),
le premier objectif (50) et le deuxième objectif (60) étant **disposés de manière décalée** dans la direction de l'axe de vision principal (18) du dispositif (10) de sorte que la première surface (70) et la deuxième surface (80) soient espacées ou décalées dans la direction parallèle à l'axe de vision principal (18) du dispositif (10),
et comprenant en outre:
un **chariot** (91) qui est mobile par rapport aux capteurs d'images (30, 40) et auquel le premier **objectif** (50) est fixé rigidement ou de manière mobile dans une direction orthogonale à l'axe optique (58) de l'objectif (50), et auquel le deuxième **objectif** (60) est fixé rigidement ;
un **module d'entraînement de chariot** (92) destiné à déplacer le chariot (91) afin de déplacer en même temps la première surface (70) et la deuxième surface (80).

7. Dispositif (10) selon l'une des revendications précédentes, dans lequel
le premier objectif (50) et le premier capteur d'images (30) ont une **première profondeur de champ** (73),
le deuxième objectif (60) et le deuxième capteur d'images (40) ont une **deuxième profondeur de champ** (83),
la première profondeur de champ (73) et la deuxième profondeur de champ (83) se **chevauchent.**

8. Dispositif (10) selon la revendication précédente, dans lequel
la **première surface** (70) est située à **l'intérieur de la deuxième profondeur de champ** (83),
la deuxième surface (80) est située à l'intérieur de la première profondeur de champ (73).

9. Dispositif (10) selon l'une des revendications 7 ou 8, dans lequel
les profondeurs de champ (73, 83) se **chevauchent** sur au moins la **moitié** de la profondeur t₁ de la première profondeur de champ (73) et au moins la moitié de la profondeur t₂ de la deuxième profondeur de champ (83) et au maximum sur **deux tiers** de la profondeur t₁ de la première profondeur de champ (73) et au maximum deux tiers de la profondeur t₂ de la deuxième profondeur de champ (83).

10. **Procédé de réglage** d'un dispositif (10) selon l'une des revendications précédentes lors de la fabrication ou de la maintenance du dispositif (10), ledit procédé comprenant les étapes suivantes :
**disposer** (101) une **jauge de réglage** (93) comprenant un premier objet (50) et un deuxième objet (60) à une position prédéterminée par rapport au dispositif (10) de sorte que le premier objet (94) soit disposé dans un premier plan prédéterminé (70) et le deuxième objet (95) soit disposé dans un deuxième plan prédéterminé (80), le premier plan (70) et le deuxième plan (80) étant chacun **orthogonaux à un axe de vision principal** (18) du dispositif (10), le premier plan prédéterminé (70) et le deuxième plan prédéterminé (80) étant parallèles l'un à l'autre et espacés l'un de l'autre ;
**régler** (102) au moins soit un premier objectif (50) soit un premier capteur d'images (30) du dispositif (10) de sorte que le premier objectif (50) génère une **image nette du premier objet** (94) sur le premier capteur d'images (30),
**régler** (103) au moins soit un deuxième objectif (60) soit un deuxième capteur d'images (40) du dispositif (10) de sorte que le deuxième objectif (60) génère une **image nette du deuxième objet** (95) sur le deuxième capteur d'images (40).
